# EUROPÄISCHE PATENTSCHRIFT

(11) **EP 4 366 727 B1**
(45) Veröffentlichungstag und Bekanntmachung des Hinweises auf die Patenterteilung: **02.04.2025**
(21) Anmeldenummer: 22761434.4
(22) Anmeldetag: 01.08.2022
(51) Int. Cl.: A61K 31/352, A61K 45/06, A61P 27/16

(54) **NEUE THERAPIEKONZEPTE FÜR DIE BEHANDLUNG VON OTITIS**
NOVEL THERAPEUTIC CONCEPTS FOR TREATING OTITIS
NOUVEAUX CONCEPTS THÉRAPEUTIQUES POUR LE TRAITEMENT DE L'OTITE

(30) Priorität: 08.10.2021 DE 102021005043; 06.12.2021 DE 102021132055
(43) Veröffentlichungstag der Anmeldung: 15.05.2024
(73) Patentinhaber: Maria Clementine Martin Klosterfrau Vertriebsgesellschaft mbH, 50670 Köln (DE)
(72) Erfinder: PLOCH, Michael, 50670 Köln (DE); BRUCHHAGE, Karl-Ludwig, 23538 Lübeck (DE); PRIES, Ralph, 23538 Lübeck (DE); LEICHTLE, Carmen, 23538 Lübeck (DE)
(74) Vertreter: Von Rohr Patentanwälte Partnerschaft mbB
(86) Internationale Anmeldenummer: PCT/EP2022/071587
(87) Internationale Veröffentlichungsnummer: WO 2023/057109

(56) Entgegenhaltungen:
- WO-A1-00/19973
- WO-A1-2022/135758
- CN-A- 109 464 428
- KR-A- 20170 039 981
- MARTINS ANITA OLIVEIRA BRITO PEREIRA BEZERRA ET AL: "Anti-edematogenic and anti-inflammatory activity of the essential oil from Croton rhamnifolioides leaves and its major constituent 1,8-cineole (eucalyptol)", BIOMEDICINE & PHARMACOTHERAPY, vol. 96, 1 December 2017 (2017-12-01), FR, pages 384 - 395, XP055981299, ISSN: 0753-3322, DOI: 10.1016/j.biopha.2017.10.005
- CHANDRAKANTHAN MADHUVANTHI ET AL: "Topical Anti-Inflammatory Activity of Essential Oils of Alpinia calcarata Rosc., Its Main Constituents, and Possible Mechanism of Action", EVIDENCE-BASED COMPLEMENTARY AND ALTERNATIVE MEDICINE, vol. 2020, 28 April 2020 (2020-04-28), US, pages 1 - 19, XP055981340, ISSN: 1741-427X, Retrieved from the Internet <URL:https://downloads.hindawi.com/journals/ecam/2020/2035671.pdf> DOI: 10.1155/2020/2035671
- CATTY ET AL: "Treating otitis media in children and infants", INTERNATIONAL JOURNAL OF AROMATHERAPY, AROMATHERAPY PUBLICATIONS, HOVE, GB, vol. 15, no. 4, 1 January 2005 (2005-01-01), pages 193 - 198, XP005178769, ISSN: 0962-4562, DOI: 10.1016/J.IJAT.2005.10.008
- ELAISSI AMEUR ET AL: "Chemical composition of essential oils of eight Tunisian Eucalyptus species and their antibacterial activity against strains responsible for otitis", BMC COMPLEMENTARY MEDICINE AND THERAPIES, BIOMED CENTRAL LTD, LONDON, UK, vol. 21, no. 1, 12 August 2021 (2021-08-12), pages 1 - 16, XP021294958, DOI: 10.1186/S12906-021-03379-Y

## Beschreibung

Die vorliegende Erfindung betrifft das technische (d. h. medizinischpharmazeutische) Gebiet der entzündlichen Erkrankungen des insbesondere menschlichen Ohres und insbesondere den Bereich der Otitiden, wie beispielsweise Otitis media (Mittelohrentzündung) bzw. Otitis externa (Außenohrentzündung), vorzugsweise Otitis media, und deren Behandlung bzw. Therapie. Insbesondere betrifft die vorliegende Erfindung einen Wirkstoff (vorliegend synonym auch als Wirksubstanz bezeichnet) sowie ein Arzneimittel und eine Zusammensetzung zur Verwendung für die Behandlung bzw. Therapie von entzündlichen Erkrankungen des menschlichen Ohres bzw. Otitiden.

Die vorliegende Erfindung betrifft im Speziellen einen Wirkstoff und ein Arzneimittel bzw. eine Zusammensetzung jeweils zur Verwendung bei der prophylaktischen und/oder therapeutischen Behandlung von entzündlichen Erkrankungen des Ohres bzw. von Otitis media mit begleitender Otorrhoe.

In entsprechender Weise betrifft die vorliegende Erfindung auch ein diesbezügliches Arzneimittel bzw. Medikament sowie eine diesbezügliche Zusammensetzung und weiterhin eine pharmazeutische Kombination auf Basis des vorliegenden Wirkstoffs.

Im Rahmen der vorliegenden Erfindung kommt dabei als Wirkstoff Cineol, vorzugsweise 1,8-Cineol, bzw. ein Cineol, vorzugsweise 1,8-Cineol, enthaltendes Arzneimittel bzw. eine diesen Wirkstoff enthaltende Zusammensetzung zum Einsatz, wie in der nachfolgenden Beschreibung und in den Patentansprüchen zu der vorliegenden Erfindung weiterführend definiert und beschrieben.

Das menschliche Ohr ist ein Sinnesorgan, mit dem Schall, wie Töne, Sprache, Laute, Geräusche oder dergleichen, aufgenommen und verarbeitet werden, wobei zum Ohr in seiner Organfunktion auch das Gleichgewichtsorgan gehört. Das menschliche Ohr kann hinsichtlich seines anatomischen Aufbaus in das Außenohr, das Mittelohr sowie das Innenohr untergliedert werden, wobei den jeweiligen Abschnitten spezielle Funktionen zukommen:
Das Außenohr umfasst u. a. die Ohrmuschel sowie den äußeren Gehörgang (Ohrkanal) und die Außenseite des Trommelfells. Das Außenohr dient zur Aufnahme bzw. dem Einfangen von Schall, wobei auch eine bestimmte Einfallsrichtung des Schalls kodiert wird, und zwar unter Einbezug der zahlreichen Erhebungen und Vertiefungen der Ohrmuschel, welche akustische Resonatoren ausbilden und welche jeweils bei Schalleinfall aus einer bestimmten Richtung differenziert angeregt werden. Auch auf dieser Basis kann eine Schallortung bzw. räumliche Schallorientierung ermöglicht werden.

Das Mittelohr umfasst u. a. das Trommelfell als solches, die Gehörknöchelchen sowie das sogenannte ovale Fenster (Eingang) und das runde Fenster (Ausgang) zum Innenohr. Die Gehörknöchelchen bilden dabei ein Hebelsystem aus, welches die vom Trommelfell aufgenommene Schallenergie über das ovale Fenster an das Innenohr weiterzugeben imstande ist. Das runde Fenster verbindet die Paukentreppe des Innenohrs mit dem Mittelohr. Das Mittelohr dient insbesondere der mechanischen Verarbeitung bzw. (Impedanz-)Wandlung des Schalls, um eine Übertragung der Schallinformation vom Außenohr auf das Innenohr zu ermöglichen. Zudem befindet sich im Mittelohr der intratympanale Anteil des Gesichtsnervs mit dem Abgang der Chorda tympani (Gesichtsnerv).

Das Innenohr umfasst u. a. die Hörschnecke, den Sacculus, den Utriculus und die Bogengänge. Im Innenohr wird das vom Mittelohr übertragene und hiervon eingehende Signal in Nervenimpulse umgesetzt. Das Gleichgewichtsorgan ist gleichermaßen Bestandteil des Innenohrs.

Der Weg des Schalls bzw. dessen Umwandlung letztendlich in entsprechende Nervensignale erfolgt dabei ausgehend von der Ohrmuschel über den Gehörgang auf das Trommelfell und anschließend über die Gehörknöchelchen, anschließend über die Hörschnecke und weiterführend über den Hörnerv, wobei der Schall auf dem Weg vom Außenohr über das Mittelohr hin zu den Nervenzellen des Innenohrs umgeformt und verarbeitet wird.

Das menschliche Gehör kann akustische Ereignisse innerhalb eines bestimmten Frequenz- und Schalldruckpegelbereichs wahrnehmen. Das Hören im Allgemeinen bzw. die auditive, aurale oder akustische Wahrnehmung stellt eine der wichtigsten Sinnesleistungen dar, so dass dem menschlichen Ohr eine bedeutende Funktion als zugrundeliegendes Sinnesorgan zukommt, und zwar auch im Hinblick auf die Aufnahme von Sprache als Mittel der Kommunikation sowie im Hinblick auf das Richtungshören (Orientierung im Raum) und darüber hinaus auch im Hinblick auf die Funktion bzw. Gewährleistung des Gleichgewichts durch das im Ohr lokalisierte Gleichgewichtsorgan.

Die obigen Ausführungen zeigen, dass es sich bei dem menschlichen Ohr um ein Organ bzw. eine funktionelle Einheit mit einem komplexen Zusammenwirken der jeweiligen Bestandteile handelt. Zur Gewährleistung der vollständigen Funktion ist es dabei insbesondere erforderlich, dass die zugrundeliegenden Strukturen nicht beeinträchtigt oder geschädigt werden, wie es beispielsweise im Fall von insbesondere entzündlichen Erkrankungen des Ohres jedoch der Fall sein kann. Derartige Erkrankungen können sogar zu einer mitunter dauerhaften und nachhaltigen Störung der funktionellen Eigenschaften des Ohres führen. Auch von daher besteht ein großer Bedarf an entsprechenden Therapien bzw. Präventionen zur Behandlung bzw. Unterbindung von insbesondere entzündlichen Erkrankungen des Ohres.

Auch vor diesem Hintergrund sind Erkrankungen des menschlichen Ohres oftmals problematisch, da diese auch zu einer mitunter drastischen Einschränkung der Funktion als Sinnesorgan und sogar zu einem vollständigen und irreversiblen Funktionsverlust führen können, einhergehend mit einer Verschlechterung bzw. sogar Unterbindung der Wahrnehmung von Schallereignissen sowie einer Beeinträchtigung der Gleichgewichtsfunktion. Zudem können Erkrankungen des Ohres auch Auswirkungen auf den gesamten Organismus haben, beispielsweise im Fall von bakteriellen Infektionen oder dergleichen, welche nämlich auch zu systemischen Erkrankungen oder Sepsis führen können.

Das menschliche Ohr kann dabei von verschiedenartigen Erkrankungsformen betroffen sein, welche zudem für den hiervon jeweils betroffenen Teil des Ohres spezifisch sind.

So ist das Außenohr durch seine relativ dünne Haut im Gehörgang und in der Ohrmuschel grundsätzlich empfänglich für Infektionen mit pathogenen Keimen, wie pathogenen Bakterien oder Pilzen, welche zu einer Entzündung des äußeren Ohres in Form einer Otitis externa führen können. Auch das Mittelohr ist insbesondere für von pathogenen Keimen bakteriell hervorgerufenen Entzündungen zugänglich, und zwar insbesondere in Form einer Mittelohrentzündung bzw. Otitis media. Durch die Entzündung können auch die Gehörknöchelchen angegriffen oder sogar zerstört werden, was mit einer nachhaltigen und irreversiblen Verringerung bzw. sogar mit einem Verlust der Hörfunktion einhergehen kann. Die Mittelohrentzündung kann auch Ausgangspunkt weiterführender Erkrankungen, wie einer Mastoiditis oder Labyrinthitis, sein. Auch das Innenohr kann von pathogenen Keimen bzw. von bakteriellen sowie viralen Infektionen befallen werden, und zwar insbesondere in Form einer Innenohrentzündung bzw. Otitis interna.

Entzündliche Erkrankungen des Ohres können gleichermaßen mit einer temporären bzw. reversiblen oder sogar dauerhaften bzw. irreversiblen Verringerung des Hörvermögens, bis hin zu einem Hörverlust, Tinnitus und Schwindel, einhergehen.

Entzündungen des Ohres bzw. Otitiden, können, wie auch zuvor angeführt, insbesondere in Abhängigkeit von dem Ort ihres Auftretens sowie ihrer weiterführenden Ausbildung bzw. Schwere differenziert werden. Insbesondere können entzündliche Erkrankungen des Ohres bzw. Otitiden auch mit einem Austritt bzw. Ausfluss von Körperflüssigkeiten bzw. Sekreten aus dem (äußeren) Gehörgang einhergehen, was im Allgemeinen als Otorrhoe bezeichnet wird und was insbesondere bei schwerem bzw. chronischem Verlauf der Erkrankung auftritt.

Eine Entzündung des Außenohres, welche als Otitis externa bezeichnet wird, stellt eine Infektion insbesondere der Gehörgangshaut dar, welche typischerweise durch pathogene Keime, insbesondere auf Basis von pathogenen Bakterien, verursacht wird. Diesbezügliche Symptome sind insbesondere Schmerzen, Ausfluss (Otorrhoe) bzw. Hörverlust, insbesondere sofern die Erkrankung mit einer Schwellung im Gehörgang einhergeht. Bei der Innenohrentzündung bzw. Otitis interna, welche gleichermaßen akut (Otitis interna acuta) oder chronisch (Otitis interna chronica) ausgebildet sein kann, handelt es sich um eine zumeist schmerzhafte Entzündung des Innenohres, wobei insbesondere das knöcherne Labyrinth mit der diesbezüglichen Hörschnecke und dem Gleichgewichtsorgan betroffen bzw. entzündet sein kann. Auch bei der Otitis interna können negative Auswirkungen auf das Hörvermögen, aber auch auf den Gleichgewichtssinn vorliegen.

Bei einer Otitis interna geht insbesondere eine Entzündung vom Mittelohr über das runde Fenster oder das ovale Fenster auf das Innenohr über. Hierbei kann es sich insbesondere auch um eine Mittelohrentzündung mit Innenohrbeteiligung handeln (insbesondere Otitis media mit Innenohrbeteiligung bzw. insbesondere Otitis media mit begleitender Innenohrentzündung). Eine derartige Erkrankung kann zu deutlichen Einschränkungen bis hin zum Funktionsverlust des Innenohres und des Gleichgewichtsorgans führen. Mögliche Folgen können eine Ertaubung oder ein Verlust des Gleichgewichtssinns sein. Ebenso kann der Gesichtsnerv sowie die hintere und mittlere Schädelgrube, insbesondere mit einer Hirnhauthautentzündung oder Hirnabszess, betroffen sein.

Was die entzündlichen Erkrankungen des Außenohres bzw. Otitis externa anbelangt, so handelt es sich hierbei beispielsweise um entzündliche Erkrankungen, welche insbesondere den äußeren Gehörgang betreffen bzw. im Bereich des äußeren Gehörgangs lokalisiert sind.

Als Mittelohrentzündung bzw. Otitis media werden im Allgemeinen Krankheitsbilder bezeichnet, welche durch eine insbesondere durch pathogene Keime hervorgerufene bzw. insbesondere bakterielle Entzündung des Mittelohrs gekennzeichnet sind, wobei nach dem Krankheitsverlauf grundsätzlich zwischen einer akuten Otitis media bzw. Otitis media acuta sowie einer chronischen Otitis media bzw. Otitis media chronica unterschieden werden kann. Die Mittelohrentzündung wird dabei insbesondere durch pathogene Keime, wie insbesondere pathogene Bakterien, verursacht, wobei die Besiedlung des Mittelohres beispielsweise über die Eustachi-Röhre aus dem Nasenraum bzw. Nasopharynx aber auch über den Blutweg erfolgen kann. Beispielsweise bei bestehender Trommelfellperforation können Krankheitserreger bzw. pathogene Keime auch von außen, beispielsweise durch Badewasser oder dergleichen, in das Mittelohr gelangen. Eine Mittelohrentzündung kann beispielsweise mit pulsierenden Ohrenschmerzen, Fieber, pochenden Ohrgeräuschen, Hörminderung, Übelkeit oder dergleichen einhergehen. Bei schweren Verläufen kann es zudem zum Austritt von Körperflüssigkeiten, wie Eiter, einer Trommelfellschädigung sowie einer nachhaltigen Beeinträchtigung des Hörvermögens kommen. Zudem kann die Entzündung bei schweren Verläufen auch auf andere Bereiche des Ohres bei schweren Verläufen übergreifen. Mittelohrentzündungen können zudem zu Vernarbungen des Trommelfells und Verwachsungen im Bereich der Gehörknöchelchen führen und somit eine bleibende Hörstörung, insbesondere in Form einer Schallleitungsschwerhörigkeit, zur Folge haben.

Zu den speziellen bzw. schweren Mittelohrentzündungen wird zudem die chronische Schleimhauteiterung bzw. Otitis media mesotympanalis bzw. Otitis media chronica mesotympanalis gezählt. Hierbei handelt es sich um eine chronische Erkrankung unter Beteiligung insbesondere der Mittelohrschleimhaut. Die Otitis media mesotympanalis geht insbesondere mit lang andauernden bzw. chronischen bzw. wiederkehrenden (rezidivierenden) Entzündungen des Mittelohrs einher, wobei oftmals auch eine bleibende Trommelfellperformation sowie insbesondere ein eitrig-schleimiger Ausfluss aus dem Gehörgang bzw. die Otorrhoe vorliegen kann.

Von der chronischen Schleimhauteiterung des Mittelohrs kann das sogenannte Cholesteatom bzw. die Otitis media epitympanalis bzw. Otitis media chronica epitympanalis abgegrenzt werden, welche(s) gleichermaßen eine schwere Form der Mittelohrentzündung bzw. Otitis media darstellt. Als Cholesteatom bzw. Otitis media epitympanalis wird im Allgemeinen eine Einwucherung von insbesondere mehrschichtig verhornendem Plattenepithel in das Mittelohr mit nachfolgender bzw. einhergehender insbesondere chronischer bzw. insbesondere eitriger Entzündung des Mittelohrs verstanden. Das Cholesteatom ist im Allgemeinen eine progrediente, destruierend-entzündliche Erkrankung bzw. Läsion des Mittelohrs, wobei aus pathologischer Sicht insbesondere eine Migration, gesteigerte Zellproliferation, Invasivität, gestörte Zelldifferenzierung, Progredienz bzw. Neigung zu Rezidiven nach operativer Intervention als diesbezügliche Charakteristika vorliegen. Das Cholesteatom ist somit insgesamt insbesondere durch einen proliferierenddestruierenden Verlauf mit möglichen Komplikationen, wie Destruktion der knöchernen Strukturen insbesondere mit Hörverlust, vestibulärer Dysfunktion, Gesichtsnervenlähmung und intrakraniellen Komplikationen, gekennzeichnet, so dass diesbezüglich insgesamt eine schwere Erkrankung des Ohres vorliegt.

Die Mittelohrentzündung bzw. Otitis media zählt in den Industrienationen zu den Erkrankungen, welche am häufigsten zu Arztbesuchen, Antibiotikaverschreibungen und Operationen führen. Entzündungen des Mittelohres bzw. Otitis media, insbesondere in der chronisch rezidivierenden Form, gehen oftmals mit einer signifikanten Verschlechterung der Lebensqualität der hiervon betroffenen Patienten einher, welche oftmals unter Ohrlaufen (Otorrhoe), Schwerhörigkeit, Sprachentwicklungsverzögerungen bis hin zur völligen Ertaubung und/oder unter Schwindel leiden. Darüber hinaus verhält sich die besonders aggressive Form der Mittelohrentzündung, nämlich das Cholesteatom bzw. Otitis media epitympanalis, gewissermaßen wie ein bösartiger Tumor, wobei die Erkrankung einer Antibiotikatherapie oftmals nicht zugänglich ist und durch fortschreitende entzündlich-destruierende Prozesse des Mittelohrs gekennzeichnet ist, wobei auch die umgebenden Strukturen betroffen bzw. zerstört werden können.

Im Allgemeinen führt die Infektion des Mittelohrs bzw. Otitis media mit pathogenen Keimen, wie insbesondere mit bakteriellen bzw. gegebenenfalls auch viralen Erregern oder pathogenen Pilzen, vorrangig bzw. zunächst zum Erkrankungsbild der insbesondere akuten Mittelohrentzündung bzw. der insbesondere akuten Otitis media, welche ein im Kindesalter häufig auftretendes Krankheitsbild darstellt. Sofern es dem Immunsystem nicht gelingt, die bakterielle Infektion mit den damit einhergehenden inflammatorischen Prozessen einzudämmen, ist eine vollständige Ausheilung der Erkrankung bzw. eine *Restitutio ad integrum* nicht gewährleistet, wobei auf den Verlauf mit der akuten Entzündung rezidivierende Episoden bzw. eine bestehende bzw. chronische Entzündung folgen kann, welche als chronische Otitis media bezeichnet wird.

Im Jahr 2004 lag die Inzidenz akuter Otitiden in Zentraleuropa nach einem Bericht der Weltgesundheitsorganisation (WHO) zur chronisch-eitrigen Mittelohrentzündung bei 36,4 pro 1.000 Einwohner, während eine Chronifizierung bzw. eine akute Otitis media etwa ein Zehntel dieser Patienten betraf, nämlich 3,69 pro 1.000 Einwohner. Dabei ist das Auftreten einer chronischen Mittelohrentzündung bei Kindern bis zum neunten Lebensjahr im Vergleich zu Erwachsenen in globaler Betrachtung um das 3-fache bis 5-fache erhöht. In Regionen mit Entwicklungsländern, wie z. B. in Zentral- und Westafrika, liegen nochmals höhere Inzidenzen vor, nämlich für das Auftreten akuter Otitiden bei 433 pro 1.000 Einwohner und für chronische Otitiden bei etwa 7 pro 1.000 Einwohner.

Eine insbesondere chronische Otitis wie eine Otitis media bzw. Otitis externa, insbesondere Otitis media, aber auch eine Otitis interna (z.B. insbesondere Otitis media mit Innenohrbeteiligung bzw. insbesondere Otitis media mit begleitender Innenohrentzündung), kann insbesondere bei speziellem bzw. schwerem Verlauf mit einer Otorrhoe, wie zuvor angeführt, und somit mit dem Austritt von Körperflüssigkeiten bzw. Sekreten aus dem äußeren Gehörgang einhergehen.

Schwere Verläufe einer Otitis und insbesondere einer Otitis media, beispielsweise wenn diese über einen Zeitraum von mehr als sechs Wochen vorliegt bzw. mit einer Otorrhoe einhergeht, können zu einer zunehmenden Destruktion des Knochengewebes und der Schleimhaut des betroffenen Mittelohrs führen. Ohne Intervention kann es dabei zu irreversiblen Schädigungen mit Trommelfelldefekten einschließlich einer Schallleitungsschwerhörigkeit kommen.

In Bezug auf schwere Verläufe einer Otitis können zentrale Trommelfelldefekte insbesondere zum Krankheitsbild der insbesondere chronischen Otitis media mesotympanalis führen, während randständige Trommelfelldefekte als insbesondere chronische Otitis media epitympanalis bzw. Cholesteatom assoziiert sein können. Schwere Verlaufsformen der Erkrankung sind dabei sehr ernst zu nehmen, da sie bei einer sich ausbreitenden unbehandelten Entzündung zu Gewebszerstörung bzw. zu einer Mastoiditis, Labyrinthitis, Fazialisparese, Meningitis, einem Hirnabszess oder zu einer Sinusvenenthrombose bis hin zum Tod führen können. Die von der Erkrankung betroffenen Patienten leiden zudem unter einer Einschränkung des Hörvermögens, welche die Alltagskommunikation massiv erschwert und sich unbehandelt progredient verschlechtert. **In** diesem Zusammenhang ist oftmals eine operative Trommelfellrekonstruktion, ein Ersatz der Gehörknöchelchenkette oder die Entfernung des Cholesteatoms unumgänglich.

Entzündliche Ohrerkrankungen und insbesondere Mittelohrentzündungen, wie Otitis media bzw. Otitis externa, aber auch Otitis externa bzw. Otitis interna (z.B. insbesondere Otitis media mit Innenohrbeteiligung bzw. insbesondere Otitis media mit begleitender Innenohrentzündung), erfordern oftmals und insbesondere bei Kindern und älteren Personen eine therapeutische Behandlung unter Einsatz von Antibiotika, insbesondere sofern die Entzündung des Mittelohres durch bakterielle und gegebenenfalls virale Erreger bzw. pathogene Keime sich nicht im selbstlimitierenden Rahmen bewegt, wie es oftmals bei Patienten mit geringerer Immunkompetenz der Fall ist. Insbesondere bei persistierender oder rezidivierender Infektion besteht im Stand der Technik somit oftmals therapeutischer Handlungsbedarf unter Anwendung entsprechender Antibiotika. Vor allem bei Kindern ist der Einsatz von Antibiotika jedoch mit strenger Indikation zu stellen und sollte immer in Abwägung der Risiken erfolgen. Die antibiotische Therapie insbesondere von Mittelohrentzündungen unter Verwendung entsprechender Antibiotika ist dabei auch insofern mit Nachteilen verbunden, als oftmals Nebenwirkungen auftreten können, wie beispielsweise Magen/Darm-Beschwerden, z. B. Durchfall, Bauchschmerzen und Übelkeit, allergische Reaktionen insbesondere der Haut, wie etwa Rötungen und/oder Juckreiz, sowie das Auftreten von Pilzinfektionen oder dergleichen. Gleichermaßen kann es unter Antibiose auch zu systemischen allergischen Reaktionen kommen, bis hin zur Ausbildung eines anaphylaktischen Schocks. Zudem kann es bei insbesondere nicht sachgemäßer Anwendung von Antibiotika auch zur Ausbildung von Resistenzen der zugrundeliegenden pathogenen Keime, insbesondere pathogenen Bakterien, führen, was die Behandlung weiterführend erschweren kann. Insbesondere können bereits resistente Keime bzw. Bakterien mit einigen Antibiotika nicht mehr zufriedenstellend bekämpft werden, so dass der Einsatz auch vor diesem Hintergrund limitiert ist.

Als pathogene Keime in Form von bakteriellen Erregern chronischer Otitiden sind beispielsweise *Pseudomonas aeruginosa, Proteus mirabilis* und *Staphylococcus aureus* sowie mitunter auch *Enterobacterales* und Anaerobier zu nennen. Ein ähnliches Erregerspektrum zeigt sich auch bei der fortgeschrittenen Entzündung mit Mastoiditis, wobei diesbezüglich insbesondere auch *Streptococcus pneumoniae* und *Haemophilus influenza* als zugrundeliegende Erreger vorliegen können.

Die Chronifizierung der Entzündung ist im Hinblick auf Otitiden multifaktorieller Genese. Insbesondere kann es sich in Bezug auf Otitiden derart verhalten, dass die Besiedlung des Ohres mit pathogenen Keimen, wie *Streptococcus pneumoniae, Haemophilus influenza* und *Staphylococcus aureus,* mit dem Ausmaß eines etwaigen Hörverlustes im Zusammenhang steht, wie es beispielsweise bei einer Otitis media mit Otorrhoe der Fall sein kann. Insbesondere kann bei Vorliegen pathogener Keime der Hörverlust stärker ausgebildet sein als ohne Vorliegen pathogener Keime. Insbesondere kann es sich in Bezug auf die pathogenen Keime im Hinblick auf Otitiden auch derart verhalten, dass diese im Bereich des betroffenen Ohres einen insbesondere pathogenen Biofilm ausbilden, wie es beispielsweise für *Pseudomonas aeruginosa* der Fall sein kann, was auch die Wirksamkeit von Antibiotika beeinflussen kann.

Die KR 2017 0039981 A betrifft eine medizinische Vorrichtung mit einem therapeutischen Mittel, welches Cineol enthält, wobei das Cineol durch Verdampfen verabreicht werden soll, und zwar im Hinblick auf eine Behandlung beispielsweise von Rhinitis oder Otitis media, wobei das Verdampfen durch Erwärmung hervorgerufen werden soll.

Das Dokument gemäß Martins et al., "Anti-edematogenic and anti-inflammatory activity of the essential oil from Croton rhamnifolioides leaves and its major constituent 1,8-cineole (eucalyptol)", Biomedicine & Pharmacotherapy, Bd. 96, 1. Dezember 2017, Seiten 384-395, betrifft Untersuchungen zur anti-ödematogenen und entzündungshemmenden Wirkung des ätherischen Öls der Blätter von Croton rhamnifolioides und seines Bestandteils 1,8-Cineol (Eucalyptol).

Weiterhin betrifft das Dokument gemäß Chandrakanthan et al., "Topical Anti-Inflammatory Acitivity of Essential Oils of Alpinia calcarata Rosc., Its Main Constituents, and Possible Mechanism of Action", Evidence-Based Complementary and Alternative Medicine, Bd. 2020, 28. April 2020, Seiten 1-19, Untersuchungen zur topischen entzündungshemmenden Wirkung der ätherischen Öle von Alpinia calcarata Rosc., deren Hauptbestandteilen und Ausführungen zu möglichen Wirkmechanismen.

Zudem betrifft das Dokument gemäß Catty et al., "Treating otitis media in children and infants", International Journal of Aromatherapy, Aromatherapy Publications, Hove, GB, Bd. 15, Nr. 4, 1. Januar 2005, Seiten 193-198, Ausführungen zur Behandlung von Otitis media bei Kindern und Säuglingen.

Die WO 00/19973 A1 betriff die Verwendung eines Boldo-Extrakts als aktives Mittel für die Zubereitung eines kosmetischen oder dermatologischen Erzeugnisses für die vorbeugende oder heilende Behandlung von Alterserscheinungen des Gewebes, für externe, lokale Anwendung für die Haut, die Nägel und/oder das Haar.

Die CN 109 464 428 A betrifft ein transdermales Pflaster, welches eine terpenbasierte pharmazeutische Zusammensetzung enthält.

Das Dokument gemäß Ameur et al., "Chemical composition of essential oils of eight Tunisian Eucalyptus species and their antibacterial activity against strains responsible for otitis", BMC Complementary Medicine and Therapies, Biomed Central Ltd, London, Bd. 21, Nr. 1, 12. August 2021, Seiten 1-16, betrifft Untersuchungen zur chemischen Zusammensetzung und zur biologischen Aktivität von ätherischen Eukalyptusölen von verschiedenen tunesischen Eukalyptusarten, wobei die antibakterielle Wirksamkeit gegen Otitis-Stämme getestet worden ist.

Die WO 2022/135758 A1 betrifft Cineol zur Verwendung bei der Verringerung pathogener Keime im menschlichen Darm bzw. bei der Reduktion der Besiedlung des menschlichen Darms mit pathogenen Keimen.

Im Lichte der obigen Ausführungen besteht somit - insbesondere auch aufgrund der mitunter großen gesundheitlichen Auswirkungen von Entzündungen des Ohres sowie der mitunter irreversiblen Folgeschäden - ein großer Bedarf nach geeigneten Therapieformen auf Basis effizienter Wirksubstanzen bzw. diesbezüglicher Zusammensetzungen und Arzneimittel, welche sich in effizienter Weise zur Behandlung von entzündlichen Erkrankungen des Ohres bzw. von Otitis, insbesondere Otitis media, eignen, wobei auch eine hohe Verträglichkeit bei gleichzeitig vorliegender hoher und spezifischer Wirksamkeit der eingesetzten Wirksubstanzen gewährleistet werden soll.

Somit liegt der vorliegenden Erfindung die Aufgabe zugrunde, ein effizientes Konzept bzw. diesbezügliche Wirkstoffe und hierauf basierende Zusammensetzungen bzw. Arzneimittel bereitzustellen, welche eine effiziente Behandlung von entzündlichen Erkrankungen des Ohres bzw. von Otitis, insbesondere Otitis media, sowie insbesondere auch der diesbezüglichen Unterformen der Erkrankungen, wie Otitis media mesotympanalis und des Cholesteatoms ermöglichen. Neben einer guten Wirksamkeit soll gleichzeitig auch eine hervorragende bzw. verbesserte Verträglichkeit und einfache Anwendung der zugrundeliegenden Wirkstoffe, Zusammensetzungen sowie Arzneimittel gewährleistet sein.

Eine wiederum weitere Aufgabe der vorliegenden Erfindung ist auch darin zu sehen, entsprechende Wirkstoffe sowie hierauf basierende Zusammensetzungen und Arzneimittel bereitzustellen, welche einer Fehlbesiedlung bzw. einer übermäßigen Besiedlung mit pathogenen Keimen bzw. nicht physiologischen Besiedlung des Ohres, insbesondere des Mittel- und/oder Außenohres, vorzugsweise des Mittelohres, aber auch des Innenohres, entgegenwirken, um auf dieser Basis die zugrundeliegende entzündliche Erkrankung abzumildern bzw. zu therapieren.

Insbesondere ist eine nochmals weitere Aufgabe der vorliegenden Erfindung auch darin zu sehen, entsprechende Wirkstoffe bzw. hierauf basierende Zusammensetzungen bzw. Arzneimittel bereitzustellen, welche grundsätzlich eine Verringerung des Einsatzes insbesondere von Antibiotika ermöglichen, so dass auch die mit Antibiotika einhergehenden Nebenwirkungen und Nachteile vermieden bzw. minimiert werden können.

Vor diesem Hintergrund ist somit eine wiederum weitere Aufgabe der vorliegenden Erfindung darin zu sehen, entsprechende Wirksubstanzen bzw. hierauf basierende Zusammensetzungen und Arzneimittel im Hinblick auf die Behandlung von entzündlichen Erkrankungen des Ohres bzw. Otitiden bereitzustellen, welche die zuvor geschilderten Nachteile des Standes der Technik zumindest weitgehend vermeiden oder aber wenigstens abschwächen.

In vollkommen überraschender Weise hat die Anmelderin nunmehr herausgefunden, dass sich Cineol, insbesondere 1,8-Cineol, unerwartet und in effizienter Weise als Wirkstoff zur prophylaktischen bzw. therapeutischen Behandlung von Otitis media eignet, und zwar im Hinblick auf solche Verlaufsformen bzw. Erkrankungen, welche mit einer Otorrhoe einhergehen bzw. hiervon begleitet werden.

Zur Lösung der zuvor geschilderten Problemstellung schlägt die vorliegende Erfindung - gemäß einem **ersten** Aspekt der vorliegenden Erfindung - Cineol, vorzugsweise 1,8-Cineol, zur Verwendung bei der prophylaktischen und/oder therapeutischen Behandlung von Otitis media mit begleitender (einhergehender) Otorrhoe gemäß Anspruch 1 vor. Jeweils vorteilhafte Weiterbildungen und Ausgestaltungen dieses Erfindungsaspekts sind Gegenstand der diesbezüglichen Unteransprüche.

Ebenfalls Gegenstand der vorliegenden Erfindung - gemäß einem **zweiten** Aspekt der vorliegenden Erfindung - ist zudem ein Arzneimittel oder Medikament zur Verwendung bei der prophylaktischen bzw. therapeutischen Behandlung von Otitis media mit begleitender (einhergehender) Otorrhoe gemäß dem diesbezüglichen unabhängigen Anspruch.

Gleichermaßen ist Gegenstand der vorliegenden Erfindung - gemäß einem **dritten** Aspekt der vorliegenden Erfindung - eine Zusammensetzung zur Verwendung bei der prophylaktischen bzw. therapeutischen Behandlung von Otitis media mit begleitender (einhergehender) Otorrhoe, gemäß dem diesbezüglichen unabhängigen Anspruch.

Schließlich ist Gegenstand der vorliegenden Erfindung - gemäß einem **vierten** Aspekt der vorliegenden Erfindung - eine pharmazeutische Kombination zur Verwendung bei der prophylaktischen bzw. therapeutischen Behandlung von Otitis media mit begleitender (einhergehender) Otorrhoe, gemäß dem diesbezüglichen unabhängigen Anspruch.

Es versteht sich von selbst, dass Ausgestaltungen, Ausführungsformen, Vorteile und dergleichen, welche nachfolgend zu Zwecken der Vermeidung von Wiederholungen zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Gegenstand der vorliegenden Erfindung - gemäß einem **ersten** Aspekt der vorliegenden Erfindung - ist somit Cineol, vorzugsweise 1,8-Cineol, zur Verwendung bei der prophylaktischen und/oder therapeutischen Behandlung von Otitis media mit begleitender (einhergehender) Otorrhoe,
wobei das Cineol systemisch appliziert wird,
wobei das Cineol als Reinstoff vorliegt und wobei das Cineol frei von anderen Terpenen ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

Diesbezüglich bezieht sich die vorliegende Erfindung insbesondere auf entzündliche Erkrankungen des menschlichen Ohres. Mit anderen Worten bezieht sich die vorliegende Erfindung insbesondere auf Cineol, vorzugsweise 1,8-Cineol, zur Verwendung bei der prophylaktischen und/oder therapeutischen Behandlung von entzündlichen Erkrankungen des menschlichen Ohres, wie zuvor angeführt.

Grundsätzlich bezieht sich die vorliegende Erfindung auch auf den diesbezüglichen Einsatz im Hinblick auf das insbesondere menschliche Innenohr.

Die von der Anmelderin im Rahmen der vorliegenden Erfindung überraschend aufgefundene, spezielle und neue wie erfinderische Anwendung bzw. medizinische Indikation für Cineol, insbesondere 1,8-Cineol, ist im Stand der Technik bislang nicht beschrieben und auch nicht erkannt worden, obwohl es sich bei Cineol, insbesondere 1,8-Cineol, an sich um einen hinlänglichen bekannten Wirkstoff handelt.

Bei dem erfindungsgemäß eingesetzten Wirkstoff Cineol, insbesondere 1,8-Cineol, handelt es sich um ein sogenanntes Terpen, insbesondere Monoterpen. Bei Terpenen handelt es sich im Allgemeinen um Naturstoffe, welche als Bestandteil der sogenannten ätherischen Öle in Form von Flüssigkeiten aus Pflanzen oder deren Bestandteilen isoliert werden können. Sie sind oftmals Duft- und Geschmacksstoffe, welche im Bereich der Lebensmittelindustrie oder der Kosmetikindustrie Anwendung finden. Daneben gewinnt jedoch auch der Einsatz von Terpenen für medizinische Zwecke an Bedeutung, da sich für eine Vielzahl von Terpenen pharmakologische Wirkungen nachweisen lassen. Terpene stellen formal Polymerisationsprodukte des Isoprens dar, wobei nach der Anzahl der Isoprenreste zwischen Monoterpenen (C₁₀-Einheiten), Sesquiterpenen (C₁₅-Einheiten), Diterpenoiden (C₂₀-Einheiten), Sesterterpenen (C₂₅-Einheiten), Triterpenen (C₃₀-Einheiten), Tetraterpenen (C₄₀-Einheiten) und Polyterpenen unterschieden wird (vgl. RÖMPP-Chemielexikon, 10. Auflage, Georg Thieme Verlag, Stuttgart/New York, 1999, Seiten 4449 und 4450, Stichwort "Terpen(oid)e"). Darüber hinaus können Terpene auch als pharmakologische Wirksubstanzen Ausgangsstoffe für die Herstellung von Arzneimitteln oder Vitaminpräparaten sowie aufgrund ihrer oftmals bakterioziden bzw. pestiziden Wirkungen in der Landwirtschaft eingesetzt werden. Speziell für eine Anzahl von Monoterpenen sind pharmakologische Wirkungen in der Bekämpfung von Krankheiten bei erfolgten systemischen Behandlungen nachgewiesen, wobei insbesondere Menthol und Cineol, insbesondere 1,8-Cineol, zu nennen sind.

Speziell der erfindungsgemäß eingesetzte Wirkstoff Cineol, insbesondere 1,8-Cineol, gehört zu den bicyclischen Epoxy-Monoterpenen, genauer gesagt zu den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und mit einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% an 1,8-Cineol), aber auch in anderen Pflanzen vor, so z. B. in Minze, Heilsalbei, Thymian, Basilikum und im Teebaum. Darüber hinaus ist 1,8-Cineol beispielsweise auch in Niaouli-, Juniperus-, Piper-, Cannabis-, Kajeput-, Salbeiöl, Myrtenöl und anderen ätherischen Ölen enthalten.

Technisch bzw. pharmazeutisch aufgereinigtes 1,8-Cineol, welches im Allgemeinen mit 99,6 %-iger bis 99,8 %-iger Reinheit vorliegen kann, wird im Allgemeinen durch fraktionierte Destillation von Eukalyptusöl gewonnen.

1,8-Cineol wird im Stand der Technik insbesondere als Expektorans bei Bronchialkatarrhen und anderen Atemwegserkrankungen, aber darüber hinaus auch als Aromastoff in der Parfümindustrie angewendet. Darüber hinaus wird 1,8-Cineol in der Zahnmedizin bei der Revision von Wurzelfüllungen verwendet. In physiologischer Hinsicht wirkt 1,8-Cineol in den oberen und unteren Atemwegen, insbesondere in der Lunge und den Nebenhöhlen, schleimlösend. 1,8-Cineol wird nach dem Stand der Technik sowohl topisch (z. B. inhalativ) als auch systemisch (z. B. in Form von Kapseln), angewendet, meist als Mischöl zusammen mit einer Vielzahl weiterer Terpene.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie die dort referierte Literatur.

Vollkommen überraschend und unerwartet ist jedoch das von der Anmelderin im Rahmen der vorliegenden Erfindung aufgefundene Wirkpotential von Cineol, vorzugsweise 1,8-Cineol, im Hinblick auf die Behandlung von Otitis media mit begleitender Otorrhoe.

In diesem Zusammenhang weist Cineol, insbesondere 1,8-Cineol, in überraschender Weise auch eine insbesondere antibiotische Wirkung gegenüber pathogenen Keimen (synonym auch als Krankheitserreger, pathogene Mikroorganismen oder Infektionserreger bezeichnet), und zwar insbesondere in Form von pathogenen Bakterien (aber auch gegenüber Pilzen bzw. Viren), auf. Hierbei handelt es sich insbesondere um solche pathogenen Keime, welche im Zusammenhang mit den entzündlichen Erkrankungen des Ohres stehen bzw. diese hervorrufen.

In diesem Zusammenhang konnte die Anmelderin in überraschender Weise auch zeigen, dass der Wirkstoff Cineol, vorzugsweise 1,8-Cineol, zu einer effizienten Reduzierung bzw. Verringerung der Keimanzahl bzw. der Keimlast pathogener Bakterien in einem von einer Otitis betroffenen Ohr führt, und zwar insbesondere was solche pathogenen Keime anbelangt, welche mit der Otitis in Verbindung stehen bzw. welche eine Otitis auszulösen imstande sind. Ohne sich auf diese Theorie festlegen oder beschränken zu wollen, kommt der Wirksubstanz Cineol, vorzugsweise 1,8-Cineol, somit im Hinblick auf die pathogenen Keime insbesondere eine antimikrobielle bzw. antibakterielle bzw. antivirale Wirkung zu. In diesem Zusammenhang werden auch mit der Otitis einhergehende entzündliche Prozesse verringert, so dass der Wirkstoff Cineol, vorzugsweise 1,8-Cineol, auch eine antientzündliche bzw. antiinflammatorische Wirkung aufweist.

Hierdurch wird im Rahmen der vorliegenden Erfindung eine effektive Linderung von Otitiden ermöglicht, und dies bei gleichzeitig hoher Verträglichkeit bzw. dem Auftreten allenfalls geringer Nebenwirkungen bei Anwendung der Wirksubstanz. In diesem Zusammenhang ist im Rahmen der vorliegenden Erfindung neben einer guten Wirksamkeit nämlich auch eine hervorragende bzw. verbesserte Verträglichkeit und einfache Anwendung des Wirkstoffs in Form von Cineol, vorzugsweise 1,8-Cineol, gewährleistet.

Insbesondere führt die Anwendung der Wirksubstanz in Form von Cineol, vorzugsweise 1,8-Cineol, im Rahmen des Erkrankungsbildes der Otitiden zu einer Verringerung der Fehlbesiedlung bzw. der Besiedlung des entzündeten Ohres mit nichtphysiologischen Keimen, wobei diesbezüglich auch eine Verbesserung der Besiedlung des Ohres mit physiologischen Keimen unterstützt bzw. hervorgerufen wird, so dass gleichermaßen auch eine Verbesserung des Keimverhältnisses im Sinne einer Verschiebung hin zu einer vermehrt physiologischen Ohrbesiedlung (d. h. Besiedlung des Ohres mit physiologisch bzw. nichtpathogenen Keimen) durch die Anwendung von Cineol, vorzugsweise 1,8-Cineol, bewirkt wird, was wiederum zu einer Abschwächung bzw. Heilung der zugrundeliegenden Erkrankung in Form der in Rede stehenden Otitiden führt.

Folglich wird im Rahmen der vorliegenden Erfindung das klinische Krankheitsbild im Hinblick auf die zugrundeliegenden Otitiden bzw. entzündlichen Erkrankungen des Ohres im Rahmen der Anwendung der Wirksubstanz Cineol, vorzugsweise 1,8-Cineol, deutlich verbessert. Dies geht gleichermaßen auch mit einer deutlichen Verbesserung der Lebensqualität der von der Erkrankung betroffenen Patienten einher, und dies auch im Hinblick auf die mit der Erkrankung einhergehenden Schmerzsymptomatik und dem diesbezüglichen Leidensdruck der Patienten.

In diesem Zusammenhang kann in Bezug auf die Behandlung von Otitis media mit begleitender (einhergehender) Otorrhoe, aber auch von Otitis interna (nämlich Otitis media mit Innenohrbeteiligung bzw. Otitis media mit begleitender Innenohrentzündung), infolge der hervorragenden Wirksamkeit von Cineol, vorzugsweise 1,8-Cineol, auch eine Eskalation des Erkrankungszustandes verhindert werden.

Was die Verwendung von Cineol, vorzugsweise 1,8-Cineol, im Hinblick auf die entzündlichen Erkrankungen des Ohres bzw. den diesbezüglichen Otitiden anbelangt, so kann im Rahmen der vorliegenden Erfindung auch eine mit den Otitiden einhergehende Otorrhoe behandelt werden, einhergehend mit einer Verbesserung der damit einhergehenden Symptome, nämlich insbesondere was eine durch Cineol, vorzugsweise 1,8-Cineol, hervorgerufene Verringerung des Austritts von Körperflüssigkeiten bzw. Sekreten aus dem Ohr bzw. dem äußeren Gehörgang anbelangt. Folglich wird einem mit der Entzündung einhergehenden "feuchten Ohr" entgegengewirkt.

Infolge der seitens der Anmelderin in völlig überraschender Weise aufgefundenen hohen therapeutischen Wirksamkeit von Cineol, vorzugsweise 1,8-Cineol, können auch verschiedene Unterformen bzw. spezielle Formen von Otitis wirksam behandelt werden, wie beispielsweise eine therapierefraktäre bzw. therapieresistente, wie antibiotikaresistente, Form der Erkrankung. Auch von daher weist die Wirksubstanz Cineol, vorzugsweise 1,8-Cineol, ein großes Wirkspektrum im Hinblick auf eine große Vielzahl entsprechend ausgebildeter Otitiden auf. Beispielsweise können auch Spezialformen der Otitis bzw. besonders schwerer Otitiden, wie Otitis media mesotympanalis oder Otitis media epitympanalis bzw. Cholesteatom, behandelt werden. Im Hinblick auf das Cholesteatom verhält es sich im Rahmen der vorliegenden Erfindung insbesondere derart, dass die zugrundeliegenden Cholesteatommassen mitunter zwar nicht bzw. nicht wesentlich aufgelöst werden (was aber weniger therapierelevant ist), jedoch der Entzündungscharakter und die Aggressivität der Entzündung nachhaltig gehemmt bzw. therapiert wird, so dass auch hier insgesamt eine sehr hohe Wirkeffizienz unter Anwendung von Cineol, vorzugsweise 1,8-Cineol, mit Verbesserung des Erkrankungszustands vorliegt.

Infolge des insgesamt positiven Einflusses von Cineol, vorzugsweise 1,8-Cineol, auf das Entzündungsgeschehen im Ohr und der hohen Wirksamkeit im Hinblick auf die Behandlung von Otitis media mit begleitender (einhergehender) Otorrhoe werden im Rahmen der vorliegenden Erfindung auch erforderliche therapeutische Operationen am Ohr ermöglicht, beispielsweise was die Implantation eines Cochleaimplantats bzw. eine Tympanoplastik bzw. Vibrant-Soundbridge-Implantation anbelangt. Denn infolge der hohen Wirksamkeit von Cineol, vorzugsweise 1,8-Cineol, werden die entzündlichen Prozesse im Ohr deutlich reduziert, und die Besiedlung des Ohres mit pathogenen Keimen wird signifikant reduziert, so dass auf dieser Basis eine Voraussetzung zur Durchführung operativer Eingriffe geschaffen wird. Cineol bzw. 1,8-Cineol kann somit im Rahmen einer präoperativen Anwendung und somit zur Vorbereitung entsprechender Ohroperationen eingesetzt werden, wobei operationsvorbereitend die zugrundeliegende Erkrankung abgeschwächt bzw. ausgeheilt wird, was den nachfolgenden operativen Eingriff erleichtert bzw. erst ermöglicht. Darüber hinaus kann auch eine perioperative oder postoperative Verabreichung von Cineol, vorzugsweise 1,8-Cineol, erfolgen, beispielsweise im Hinblick auf die Vermeidung des Auftretens von Rezidiven oder dergleichen. Durch die Verabreichung von Cineol, vorzugsweise 1,8-Cineol, und der damit einhergehenden Verringerung der Anzahl pathogener Keime im von einer Entzündungserkrankung bzw. Otitis betroffenen Ohr kann zudem auch eine systemische Infektion im Rahmen der Operation bzw. im Nachgang zu einer durchgeführten Operation vermieden bzw. die diesbezügliche Wahrscheinlichkeit deutlich verringert werden.

Infolge der überraschend gefundenen Wirksamkeit von Cineol, vorzugsweise 1,8-Cineol, kann vorliegend gegebenenfalls auf die Verabreichung von Antibiotika mitunter vermieden bzw. der diesbezügliche Einsatz verringert werden. Folglich können im Rahmen der vorliegenden Erfindung die mit der Verabreichung von Antibiotika einhergehenden Nachteile, wie spezifische Nebenwirkungen und die Ausbildung von Resistenzen, überkommen bzw. vermieden werden.

Insbesondere wird erfindungsgemäß ein neuer Therapieansatz auch im Hinblick auf eine Regulation bzw. Einflussnahme auf insbesondere chronifizierte und in Zusammenhang mit entzündlichen Erkrankungen des Ohres stehende Entzündungsreaktionen bzw. -prozesse bereitgestellt, und dies, ohne eine antibiotikainduzierte Resistenz der pathogenen keime bzw. pathogenen Mikroorganismen zu provozieren. Dabei können insbesondere auch vulnerable Gruppen, wie (Klein-)Kinder, mit dem erfindungsgemäßen Konzept therapiert werden.

Aufgrund der antimikrobiellen Wirksamkeit gegen pathogene Keime kann die im Rahmen der vorliegenden Erfindung angeführte Verabreichung von Cineol, vorzugsweise 1,8-Cineol, auch zu einer Bekämpfung bzw. einem Abbau bzw. zu einem "Aufbrechen" von durch pathogene Keime gebildeten bzw. hervorgerufenen pathogenen Biofilmen im Ohr führen, so dass auch auf dieser Basis die Besiedlung des Ohres mit pathogenen Keimen verringert wird und zudem die Zugänglichkeit etwaig weiterer Wirkstoffe, wie Antibiotika oder dergleichen, in Bezug auf den Wirkort am bzw. im Ohr verbessert wird (so dass deren Wirkung verstärkt bzw. deren Menge verringert werden kann). Dabei kann das Cineol, insbesondere 1,8-Cineol, die in Rede stehenden Biofilme - ohne sich auf diese Theorie beschränken oder berufen zu wollen - sozusagen infiltrieren bzw. durchdringen, so dass es dort zu einer Entfaltung seiner Wirkung kommen kann.

Die aufgezeigte Wirksamkeit von Cineol, insbesondere 1,8-Cineol, ist dabei umso überraschender, als die zugrundeliegenden entzündlichen Prozesse und deren Chronifizierung im Hinblick auf Otitiden multifaktorieller Genese sind, wie zuvor angeführt.

Insgesamt wird im Rahmen der vorliegenden Erfindung somit ein effektives Konzept im Hinblick auf die Behandlung von entzündlichen Erkrankungen des Ohres bzw. von Otitis media mit begleitender Otorrhoe unter Einsatz von Cineol bzw. 1,8-Cineol als zugrundeliegende Wirksubstanz bereitgestellt.

Wie zuvor angeführt, fokussiert die vorliegende Erfindung auf die Behandlung von Otitis, und zwar von Otitis media mit begleitender bzw. hiermit einhergehender Otorrhoe.

Erfindungsgemäß kann grundsätzlich auch eine Behandlung von entzündlichen Erkrankungen des Innenohrs bzw. Otitis interna (nämlich Otitis media mit Innenohrbeteiligung bzw. Otitis media mit begleitender Innenohrentzündung) erfolgen, wie zuvor angeführt.

Insbesondere verhält es sich im Rahmen der vorliegenden Erfindung derart, dass der Wirkstoff Cineol, vorzugsweise 1,8-Cineol, zur Verwendung bei der prophylaktischen bzw. therapeutischen Behandlung von Otitis media mit begleitender (einhergehender) Otorrhoe eingesetzt wird.

Erfindungsgemäß verhält es sich derart, dass der Wirkstoff Cineol, vorzugsweise 1,8-Cineol, zur Verwendung bei der prophylaktischen und/oder therapeutischen Behandlung von Otitis media mit begleitender (einhergehender) Otorrhoe eingesetzt wird.

Wie zuvor angeführt, weist der Wirkstoff Cineol, vorzugsweise 1,8-Cineol, auch eine Wirkung gegenüber pathogenen Keimen im Ohr auf: Vor diesem Hintergrund betrifft die vorliegende Erfindung gemäß dem vorliegenden Aspekt auch Cineol, vorzugsweise 1,8-Cineol, insbesondere wie zuvor definiert, zur Verwendung bei der Reduktion der Last (Keimlast) und/oder der Anzahl (Keimanzahl) von im Mittelohr befindlichen und/oder lokalisierten pathogenen Keimen, insbesondere pathogenen Bakterien, bei der prophylaktischen und/oder therapeutischen Behandlung von Otitis media mit begleitender (einhergehender) Otorrhoe.

Im Hinblick auf die vorangehenden und nachfolgenden Ausführungen verhält es sich insbesondere derart, dass der Anteil bzw. Keimanteil der pathogenen Keime auf die Gesamtanzahl an Keimen (Gesamtkeimanzahl) im Ohr, insbesondere im Mittel- und/oder Außenohr (insbesondere äußerer Gehörgang des Außenohrs), vorzugsweise im Mittelohr, bezogen ist (insbesondere so dass die Gesamtanzahl an Keimen die heranzuziehende Bezugsgröße darstellt). In entsprechender Weise verhält es sich zudem insbesondere derart, dass der vorliegend angeführte Anteil an pathogenen Bakterien auf die Gesamtanzahl an Bakterien im Ohr, insbesondere im Mittel- und/oder Außenohr, vorzugsweise im Mittelohr, bezogen ist. Bei den entsprechenden Anteilen handelt es sich somit um relative Werte mit Bezugnahme auf die zuvor angeführte Gesamtanzahl an Bakterien bzw. Keimen.

Die vorliegende Offenbarung (nicht Teil der beanspruchten Erfindung) betrifft gemäß dem vorliegenden Aspekt weiterhin auch Cineol, vorzugsweise 1,8-Cineol, insbesondere wie zuvor definiert, zur Verwendung bei der Reduktion und/oder beim Abbau und/oder der Zerstörung eines pathogenen Biofilms im Ohr, insbesondere im Mittel- und/oder Außenohr (insbesondere im äußeren Gehörgang des Außenohrs), vorzugsweise im Mittelohr, insbesondere wobei der Biofilm von pathogenen Keimen, insbesondere pathogenen Bakterien, gebildet ist, bei der prophylaktischen und/oder therapeutischen Behandlung von Otitis media mit begleitender (einhergehender) Otorrhoe.

Folglich stellt die vorliegende Erfindung im Hinblick auf den Wirkstoff Cineol, vorzugsweise 1,8-Cineol, insbesondere auch auf eine Reduktion bzw. einen Abbau bzw. eine Zerstörung von pathogenen Biofilmen im Ohr ab. Dabei verhält es sich im Rahmen der vorliegenden Erfindung insbesondere derart, dass die pathogenen Biofilme im Zusammenhang mit Otitis media mit begleitender (einhergehender) Otorrhoe, stehen bzw. die diesbezüglichen Entzündungen bzw. Erkrankungen verursachen, und zwar insbesondere auf Basis zugrundeliegender pathogener Keime bzw. Bakterien. Insbesondere werden die pathogenen Biofilme dabei von solchen pathogenen Keimen, insbesondere pathogenen Bakterien, gebildet, welche im Zusammenhang mit den Entzündungen im Ohr bzw. im Zusammenhang mit den Otitiden stehen bzw. diese hervorrufen.

Erfindungsgemäß ist insbesondere vorgesehen, dass das Cineol, vorzugsweise 1,8-Cineol, zur Verwendung nach einem der vorangehenden Ansprüche, zur Reduktion der Last (Keimlast) und/oder der Anzahl (Keimanzahl) von im Mittelohr befindlichen und/oder lokalisierten pathogenen Keimen, insbesondere pathogenen Bakterien, eingesetzt oder verwendet wird.

Zudem kann im Rahmen der vorliegenden Erfindung das Cineol, vorzugsweise 1,8-Cineol, zur Reduktion des Anteils (Keimanteils) von im Mittelohr befindlichen und/oder lokalisierten pathogenen Keimen, insbesondere pathogenen Bakterien, eingesetzt oder verwendet werden.

Was die in Rede stehenden pathogenen Keime anbelangt, so kann es sich im Rahmen der vorliegenden Erfindung insbesondere wie folgt verhalten:
Insbesondere sind die pathogenen Keime ausgewählt aus der Gruppe von die Otitis media mit begleitender (einhergehender) Otorrhoe verursachenden und/oder verstärkenden und/oder hiermit im Zusammenhang stehenden Keimen.

Erfindungsgemäß sind die pathogenen Keime zudem insbesondere ausgewählt aus der Gruppe von die Otitis media mit begleitender (einhergehender) Otorrhoe verursachenden und/oder verstärkenden und/oder hiermit im Zusammenhang stehenden Bakterien.

Weiterführend verhält es sich im Rahmen der vorliegenden Erfindung insbesondere derart, dass die pathogenen Keime ausgewählt sind aus der Gruppe von Bakterien der Gattung *Pseudomonas, Proteus, Staphylococcus, Haemophilus, Streptococcus, Stenotrophomonas, Enterococcus* und *Moraxella* sowie deren Kombinationen, insbesondere aus der Gruppe von Bakterien der Gattung *Pseudomonas, Proteus* und *Staphylococcus* sowie deren Kombinationen, vorzugsweise aus der Gruppe von Bakterien der Gattung *Pseudomonas* und *Proteus* sowie deren Kombinationen.

In diesem Zusammenhang kann es insbesondere vorgesehen sein, dass die pathogenen Keime ausgewählt sind aus der Gruppe von Bakterien der Spezies *Pseudomonas aeruginosa, Pseudomonas stutzeri, Proteus mirabilis, Staphylococcus aureus, Haemophilus influenza, Streptococcus pneumoniae, Streptococcus pyogenes, Stenotrophomonas maltophila, Enterococcus faecalis* und *Moraxella catarrhalis* sowie deren Kombinationen, insbesondere aus der Gruppe von Bakterien der Spezies *Pseudomonas aeruginosa, Proteus mirabilis* und *Staphylococcus aureus* sowie deren Kombinationen, vorzugsweise von Bakterien der Spezies *Pseudomonas aeruginosa* und *Proteus mirabilis* sowie deren Kombinationen.

Gegenüber den vorgenannten Bakteriengattungen bzw. -spezies liegt eine besonders hohe Wirksamkeit von Cineol bzw. 1,8-Cineol vor. Auf dieser Basis kann eine effektive Behandlung der zugrundeliegenden entzündlichen Erkrankungen des Ohres bzw. der Otitiden erfolgen. In diesem Zusammenhang besteht insbesondere - ohne sich auf diese Theorie beschränken oder berufen zu wollen - ein unmittelbarer Zusammenhang der zuvor angeführten speziellen pathogenen Bakteriengattungen bzw. -spezies im Hinblick auf das Entstehen bzw. Vorliegen der Otitis media mit begleitender (einhergehender) Otorrhoe, und zwar insbesondere insofern, als die Erkrankung durch die in Rede stehenden pathogenen Keime hervorgerufen (d. h. die Keime ursächlich für die Erkrankung sind) oder dass die in Rede stehenden Keime mit der Erkrankung einhergehen bzw. diese aufrechterhalten bzw. weiterführend verschlimmern.

Was die pathogenen Keime zudem anbelangt, so kann es sich im Rahmen der vorliegenden Erfindung grundsätzlich auch derart verhalten, dass die pathogenen Keime ausgewählt sind aus der Gruppe von Pilzen, wie Schimmelpilzen, und Hefen, insbesondere aus der Gruppe der Gattung *Aspergillus* und *Candida,* sowie deren Kombinationen. Beispielsweise kann es sich bei den pathogenen Keimen um *Aspergillus spp.* bzw. *Candida parapilosis* handeln. Grundsätzlich können die pathogenen Keime erfindungsgemäß auch aus der Gruppe der Viren ausgewählt sein, wobei es sich hierbei insbesondere um im Zusammenhang mit den vorgenannten entzündlichen Erkrankungen des Ohres bzw. den vorgenannten Otitiden stehende bzw. die zugrundeliegenden entzündlichen Erkrankungen hervorrufende Viren handelt.

Im Rahmen der vorliegenden Erfindung wird unter Verwendung von Cineol, vorzugsweise 1,8-Cineol, insbesondere auch eine Verbesserung bzw. Unterstützung der natürlichen bzw. physiologischen (Keim-)Flora im Ohr ermöglicht, was den zugrundeliegenden entzündlichen Erkrankungen bzw. Otitiden gleichermaßen entgegenwirkt. Unter Verwendung von Cineol, insbesondere 1,8-Cineol, kann es sich im Rahmen der vorliegenden Erfindung somit insbesondere auch derart verhalten, dass die physiologische (Keim-)Flora im Ohr, insbesondere im Mittelohr und/oder im Außenohr (insbesondere im äußeren Gehörgang), vorzugsweise im Mittelohr, verbessert bzw. unterstützt wird (d. h. insbesondere die Anzahl an physiologischen Keimen bzw. deren Anteil an der Gesamtflora bzw. an der Gesamtanzahl an Keimen erhöht wird).

Somit kann es sich erfindungsgemäß insbesondere derart verhalten, dass das Cineol, vorzugsweise 1,8-Cineol, zur Verwendung, insbesondere wie zuvor definiert, des Weiteren und/oder zusätzlich und/oder gleichermaßen zur Erhöhung, Vermehrung oder Unterstützung von im Ohr, insbesondere im Mittel- und/oder Außenohr, vorzugsweise im Mittelohr, befindlichen und/oder lokalisierten physiologischen Keimen (nichtpathogenen Keimen), insbesondere physiologischen Bakterien (nichtpathogenen Bakterien), verwendet oder eingesetzt wird.

Gleichermaßen kann es sich erfindungsgemäß derart verhalten, dass das Cineol, vorzugsweise 1,8-Cineol, des Weiteren und/oder zusätzlich und/oder gleichermaßen zur Erhöhung der Beladung (Keimbeladung) oder der Anzahl (Keimanzahl) oder des Anteils (Keimanteil) von im Ohr, insbesondere im Mittel- und/oder Außenohr, vorzugsweise im Mittelohr, befindlichen und/oder lokalisierten physiologischen Keimen (nichtpathogenen Keimen), insbesondere physiologischen Bakterien (nichtpathogenen Bakterien), verwendet oder eingesetzt wird.

Zudem kann es erfindungsgemäß auch vorgesehen sein, dass das Cineol, vorzugsweise 1,8-Cineol, des Weiteren und/oder zusätzlich und/oder gleichermaßen zur Erhöhung der Besiedlung des Ohres, insbesondere des Mittel- und/oder Außenohres, vorzugsweise des Mittelohres, mit physiologischen Keimen (nichtpathogenen Keimen), insbesondere physiologischen Bakterien (nichtpathogenen Bakterien), verwendet oder eingesetzt wird.

Wie zuvor angeführt, können im Rahmen der vorliegenden Erfindung zahlreiche Formen bzw. Unterformen von Otitis behandelt werden.

Gemäß dem vorliegenden Aspekt betrifft die vorliegende Erfindung in diesem Zusammenhang gleichermaßen auch Cineol, vorzugsweise 1,8-Cineol, wie zuvor definiert,
zur Verwendung bei der prophylaktischen und/oder therapeutischen Behandlung von Otitis media mit begleitender (einhergehender) Otorrhoe, wobei die Otitis eine therapierefraktäre und/oder therapieresistente, insbesondere medikamenten- und/oder arzneimittelresistente, vorzugsweise antibiotikaresistente, Otitis ist.

Somit kann es sich erfindungsgemäß insbesondere derart verhalten, dass die Erkrankung eine therapierefraktäre und/oder therapieresistente, insbesondere medikamenten- und/oder arzneimittelresistente, vorzugsweise antibiotikaresistente, Erkrankung ist.

**In** entsprechender Weise kann es sich erfindungsgemäß auch derart verhalten, dass die Otitis eine therapierefraktäre und/oder therapieresistente, insbesondere medikamenten- und/oder arzneimittelresistente, vorzugsweise antibiotikaresistente, Otitis ist.

Insbesondere kann es sich erfindungsgemäß derart verhalten, dass die pathogenen Keime, insbesondere die mit den entzündlichen Erkrankungen des Ohres und/oder den Otitiden in Zusammenhang stehenden oder diese verursachenden pathogenen Keime, therapierefraktäre und/oder therapieresistente, insbesondere medikamenten- und/oder arzneimittelresistente, vorzugsweise antibiotikaresistente, pathogene Keime sind. Vorzugsweise kann es sich bei den pathogenen Keimen um medikamenten- und/oder arzneimittelresistente, insbesondere antibiotikaresistente, pathogene Bakterien handeln. Denn auch diesbezüglich konnte seitens der Anmelderin eine hohe Wirksamkeit von Cineol, insbesondere 1,8-Cineol, aufgezeigt werden. Bei den pathogenen Keimen kann es sich dabei auch um sogenannte MRGN-Bakterien (multiresistente gramnegative Bakterien) handeln, beispielsweise 3MRGN-Bakterien (Resistenz gegenüber drei Antibiotikaklassen) oder 4MRGN-Bakterien (Resistenz gegenüber vier Antibiotikaklassen) handeln.

Weiterhin kann die Otitis eine akute Otitis oder chronische Otitis, insbesondere eine chronische Otitis, vorzugsweise eine chronisch polypöse Otitis oder eine chronisch granuläre Otitis, sein. Zudem kann die Otitis auch eine rezidivierende Otitis oder chronisch-rezidivierende Otitis, insbesondere eine chronische Otitis, vorzugsweise eine chronisch polypöse Otitis oder eine chronisch granuläre Otitis, sein. Erfindungsgemäß kann es insbesondere vorgesehen sein, dass die Otitis eine rezidivierende Otitis media mit begleitender (einhergehender) Otorrhoe ist.

Erfindungsgemäß handelt es sich im Hinblick auf die entzündlichen Erkrankungen des Ohres um Otitis media mit begleitender (einhergehender) Otorrhoe. Insbesondere kann es sich um eine foetide Otorrhoe handeln. Insbesondere kann es erfindungsgemäß der Fall sein, dass die Otitis eine chronische Otitis media mit begleitender (einhergehender) Otorrhoe ist.

Weiterhin kann es erfindungsgemäß der Fall sein, dass die Otitis eine Otitis media epitympanalis (Cholesteatom bzw. Otitis media epitympanalis mit Cholesteatom) oder eine Otitis media mesotympanalis, vorzugsweise eine Otitis media epitympanalis, ist. Diesbezüglich kann es sich insbesondere um chronische Formen handeln (d. h. Otitis media chronica epitympanalis bzw. Otitis media chronica mesotympanalis). Auch die vorgenannten Otitiden sind dabei von einer Otorrhoe begleitet bzw. gehen hiermit einher (d. h. Otitis media (chronica) epitympanalis mit begleitender Otorrhoe bzw. Otitis media (chronica) mesotympanalis mit begleitender Otorrhoe).

Weiterhin kann es sich erfindungsgemäß derart verhalten, dass die Otitis media mit begleitender (einhergehender) Otorrhoe, bevorzugt Otitis media epitympanalis, mit einer Entzündungsmediation, insbesondere einer Heraufregulation und/oder einer erhöhten Produktion oder einer erhöhten Freisetzung von insbesondere proinflammatorischen Zytokinen, vorzugsweise TNF-alpha (Tumornekrosefaktor-alpha) und/oder Interleukin-1 (IL-1), bevorzugt TNF-alpha, einhergeht oder hiermit im Zusammenhang steht.

Diesbezüglich kann es insbesondere vorgesehen sein, dass das Cineol, vorzugsweise 1,8-Cineol, des Weiteren und/oder zusätzlich und/oder gleichermaßen zur Suppression oder Abschwächung einer im Rahmen der Otitis media mit begleitender (einhergehender) Otorrhoe, bevorzugt Otitis media epitympanalis, auftretenden Entzündungsmediation verwendet oder eingesetzt wird.

Insbesondere kann es sich im Rahmen der vorliegenden Erfindung derart verhalten, dass das Cineol, vorzugsweise 1,8-Cineol, eine supprimierende oder abschwächende Wirkung gegenüber einer im Rahmen der Otitis media mit begleitender (einhergehender) Otorrhoe, bevorzugt Otitis media epitympanalis, auftretenden Entzündungsmediation ausübt. Diesbezüglich kann es sich erfindungsgemäß insbesondere derart verhalten, dass das Cineol, vorzugsweise 1,8-Cineol, eine Herabregulation und/oder eine verringerte Produktion und/oder eine verringerte Ausschüttung von insbesondere proinflammatorischen Zytokinen, vorzugsweise TNF-alpha (Tumornekrosefaktor-alpha bzw. TNF-α) und/oder Interleukin-1beta (IL-1β), bevorzugt TNF-alpha, bewirkt bzw. hervorruft.

Insbesondere kann das Cineol, vorzugsweise 1,8-Cineol, somit zur Herabregulation und/oder zur Verringerung der Produktion und/oder zur Verringerung der Ausschüttung von insbesondere proinflammatorischen Zytokinen, vorzugsweise TNF-alpha (Tumornekrosefaktor-alpha) und/oder Interleukin-1beta (IL-1β), bevorzugt TNF-alpha, verwendet oder eingesetzt werden.

In diesem Zusammenhang verhält es sich im Hinblick auf die zugrundeliegenden Erkrankungen insbesondere in Form des Cholesteatoms insbesondere derart, dass - ohne sich auf diese Theorie beschränken oder berufen zu wollen - die vorgenannten proinflammatorischen Zytokine, vorzugsweise TNF-alpha, die Entzündung und/oder die Apoptose regulieren bzw. beeinflussen und eine wichtige Rolle auch im Hinblick auf die Chronifizierung der Erkrankung spielen. Durch die Wirkung von Cineol, vorzugsweise 1,8-Cineol, kann somit auch diesen Prozessen entgegengewirkt werden.

Insbesondere auch in diesem Zusammenhang kann dem Wirkstoff Cineol, vorzugsweise 1,8-Cineol, im Hinblick auf die erfindungsgemäße Verwendung mit der Behandlung entzündlicher Erkrankungen des Ohres bzw. von Otitiden auch eine antiinflammatorische Wirkung zugesprochen werden. Folglich werden auch auf dieser Basis entzündliche Prozesse, welche im Zusammenhang mit den entzündlichen Erkrankungen des Ohres bzw. der Otitiden stehen, verringert bzw. herabreguliert, so dass auch auf dieser Basis ein positiver Effekt bzw. ein Wirkeffekt im Hinblick auf die Behandlung der angeführten entzündlichen Erkrankungen des Ohres bzw. der Otitiden vorliegt.

Insbesondere kann es sich erfindungsgemäß folglich derart verhalten, dass das Cineol, vorzugsweise 1,8-Cineol, des Weiteren und/oder zusätzlich und/oder gleichermaßen als antiinflammatorischer Wirkstoff, insbesondere als lokal und/oder systemisch wirkender antiinflammatorischer Wirkstoff, verwendet oder eingesetzt wird.

Darüber hinaus kann es erfindungsgemäß auch vorgesehen sein, dass das Cineol, vorzugsweise 1,8-Cineol, des Weiteren und/oder zusätzlich und/oder gleichermaßen zur Vorbeugung und/oder Verhinderung und/oder zur prophylaktischen und/oder therapeutischen Behandlung von mit Otitis media mit begleitender (einhergehender) Otorrhoe im Zusammenhang stehenden und/oder hierdurch hervorgerufenen Folge- und/oder Begleiterkrankungen, insbesondere ausgewählt aus der Gruppe von Mastoisitis, Labyrinthitis, Hirnhautentzündung, Sinusvenenthrombose, Sepsis, Gesichtsnervenlähmung und Tinnitus sowie deren Kombinationen, verwendet oder eingesetzt wird. Im Rahmen der vorliegenden Erfindung können somit mitunter schwerwiegende Folge- bzw. Begleiterkrankungen von Otitis media mit begleitender Otorrhoe insbesondere im Sinne einer Prophylaxe verhindert werden oder begleitend zu einer zugrundeliegenden Otitis mitbehandelt bzw. co-therapiert werden.

Darüber hinaus kann das Cineol, vorzugsweise 1,8-Cineol, auch im Hinblick auf die Durchführung von Ohroperationen, insbesondere Operationen des Mittel- und/oder Außenohres, vorzugsweise des Mittelohres, eingesetzt bzw. verwendet werden, und zwar insbesondere wie folgt: Erfindungsgemäß kann das Cineol, vorzugsweise 1,8-Cineol, präoperativ und/oder zur präoperativen Vorbereitung und/oder zur Ermöglichung von chirurgischen Eingriffen (chirurgischen Operationen), insbesondere therapeutisch-chirurgischen Eingriffen, am Ohr (Ohroperationen), insbesondere am Mittel- und/oder Außenohr (Mittel- und/oder Außenohroperationen), vorzugsweise am Mittelohr (Mittelohroperationen), wie tympanoplastischen Operationen und/oder Implantationsoperationen (Implantation), verwendet oder eingesetzt werden.

Zudem kann das Cineol, vorzugsweise 1,8-Cineol, im Rahmen der vorliegenden Erfindung postoperativ und/oder zur postoperativen Nachbehandlung von chirurgischen Eingriffen (chirurgischen Operationen), insbesondere therapeutisch-chirurgischen Eingriffen, am Ohr (Ohroperationen), insbesondere am Mittel- und/oder Außenohr (Mittel- und/oder Außenohroperationen), vorzugsweise am Mittelohr (Mittelohroperationen), wie tympanoplastischen Operationen und/oder Implantationsoperationen (Implantation), verwendet oder eingesetzt werden. Somit kann erfindungsgemäß des Weiteren und/oder zusätzlich und/oder gleichermaßen ein präoperativer und/oder postoperativer, vorzugsweise präoperativer, Einsatz von Cineol, vorzugsweise 1,8-Cineol, erfolgen.

Weiterhin kann das Cineol, vorzugsweise 1,8-Cineol, im Rahmen der vorliegenden Erfindung intraoperativ und/oder während chirurgischer Eingriffe, insbesondere therapeutisch-chirurgischen Eingriffen, am Ohr (Ohroperationen), insbesondere am Mittel- und/oder Außenohr, vorzugsweise am Mittelohr (Mittelohroperationen), wie tympanoplastischen Operationen und/oder Implantationsoperationen (Implantation), verwendet oder eingesetzt werden. Somit kann erfindungsgemäß des Weiteren und/oder zusätzlich und/oder gleichermaßen ein intraoperativer Einsatz von Cineol, vorzugsweise 1,8-Cineol, erfolgen.

Insgesamt kann erfindungsgemäß des Weiteren und/oder zusätzlich gleichermaßen ein perioperativer Einsatz von Cineol, vorzugsweise 1,8-Cineol, erfolgen.

Erfindungsgemäß kann der Wirkstoff Cineol, vorzugsweise 1,8-Cineol, auch im Hinblick auf die Behandlung von Otitis media mit Innenohrbeteiligung bzw. insbesondere Otitis media mit begleitender Innenohrentzündung eingesetzt werden:
Diesbezüglich betrifft die vorliegende Erfindung gemäß dem vorliegenden Aspekt somit auch Cineol, vorzugsweise 1,8-Cineol, insbesondere wie zuvor definiert, zur Verwendung bei der prophylaktischen und/oder therapeutischen Behandlung von Otitis media mit begleitender Innenohrentzündung und mit begleitender (einhergehender) Otorrhoe.

Folglich kann es sich erfindungsgemäß derart verhalten, dass die Otitis eine eine Otitis media mit begleitender Innenohrentzündung und mit begleitender (einhergehender) Otorrhoe ist.

Wie zuvor angeführt, fokussiert die vorliegende Erfindung auf entzündliche Erkrankungen des Ohres. Erfindungsgemäß kann es sich diesbezüglich insbesondere derart verhalten, dass das Cineol nicht zur Verwendung bei der Verringerung bei der Verringerung pathogener Keime bzw. pathogener Erreger im menschlichen Darm, insbesondere in der menschlichen Darmflora, vorgesehen ist bzw. von dieser Verwendung ausgenommen ist.

Insbesondere kann es sich erfindungsgemäß derart verhalten, dass Cineol, vorzugsweise 1,8-Cineol, zur Verwendung bei der Reduktion der Besiedlung des menschlichen Darms, insbesondere der menschlichen Darmflora, mit pathogenen Keimen bzw. pathogenen Erregern, ausgenommen ist. Folglich kann es erfindungsgemäß insbesondere vorgesehen sein, dass vorliegend der Wirkstoff Cineol, vorzugsweise 1,8-Cineol, von der Verwendung bei der Reduktion der Besiedlung des menschlichen Darms, insbesondere der menschlichen Darmflora, mit pathogenen Keimen bzw. pathogenen Erregern, ausgenommen ist. Hierbei kann es sich insbesondere auch um für eine Fehlbesiedlung der Darmflora typische pathogene Keime bzw. pathogene Erreger handeln, d. h. insbesondere solchen pathogenen Keimen bzw. pathogenen Erregern, die insbesondere speziell im Zusammenhang mit einer Fehlbesiedlung des Darms, insbesondere menschlichen Darms, stehen.

Erfindungsgemäß kann es sich somit insbesondere derart verhalten, dass Cineol, vorzugsweise 1,8-Cineol, nicht zur Verwendung bei der Reduktion der Besiedlung des menschlichen Darms, insbesondere der menschlichen Darmflora, mit pathogenen Keimen bzw. pathogenen Erregern eingesetzt wird.

Gemäß der vorliegenden Erfindung gemäß diesem Erfindungsaspekt ist es vorgesehen, dass das Cineol, vorzugsweise 1,8-Cineol, systemisch, insbesondere peroral oder parenteral, vorzugsweise peroral, appliziert wird und/oder dass das Cineol, vorzugsweise 1,8-Cineol, zur systemischen, insbesondere peroralen oder parenteralen, vorzugsweise peroralen, Applikation hergerichtet ist. Auf diese Weise werden hohe systemische Wirkdosen bzw. Wirkstoffpegel (d. h. Wirkstoffspiegel bzw. -konzentrationen) erreicht, und dies auch am Wirkort selbst (d. h. im Bereich des Ohres, insbesondere im Bereich des Mittel- bzw. Außenohres, vorzugsweise im Bereich des Mittelohres), so dass eine besonders gute Wirksamkeit bzw. Effizienz realisiert werden kann.

Mit anderen Worten kann es sich erfindungsgemäß insbesondere derart verhalten, dass das Cineol, vorzugsweise 1,8-Cineol, in Form einer systemisch, insbesondere peroral oder parenteral, vorzugsweise peroral, zu verabreichenden Darreichungsform appliziert wird.

Gemäß einer vorteilhaften Ausführungsform nach diesem Erfindungsaspekt ist es insbesondere vorgesehen, dass das Cineol, vorzugsweise 1,8-Cineol, in Form einer peroral zu verabreichenden Darreichungsform appliziert wird bzw. dass das Cineol, vorzugsweise 1,8-Cineol, in einer peroral zu verabreichenden Darreichungsform hergerichtet ist.

Gemäß einer wiederum besonderen Ausführungsform der vorliegenden Erfindung gemäß diesem Aspekt kann es insbesondere vorgesehen sein, dass das Cineol, vorzugsweise 1,8-Cineol, als magensaftresistente, aber dünndarmlösliche systemische Darreichungsform, bevorzugt als Kapsel, Dragee, Pille, Tablette oder dergleichen, appliziert wird und/oder dass das Cineol, vorzugsweise 1,8-Cineol, zur Verabreichung als magensaftresistente, aber dünndarmlösliche systemische Darreichungsform, bevorzugt Kapsel, Dragee, Pille, Tablette oder dergleichen, hergerichtet ist.

Für die systemische Anwendung von Cineol, insbesondere 1,8-Cineol, sind im Handel verschiedene Präparate, insbesondere auf Basis von im Allgemeinen magensaftresistenten, aber dünndarmlöslichen Darreichungsformen bzw. Kapseln, verfügbar.

Der Vorteil derartiger Darreichungsformen ist, dass zum einen der Wirkstoff Cineol, insbesondere 1,8-Cineol, langzeitstabil vorliegt und zum anderen besonders hohe systemische Wirkstoffkonzentrationen bzw. Wirkstoffpegel erreicht werden können. Auf diese Weise können auch besonders hochdosierte Darreichungsformen bereitgestellt werden, so dass eine genaue und hohe Dosierung des Wirkstoffs ermöglicht wird.

Im Rahmen der vorliegenden Erfindung wird das Cineol, insbesondere 1,8-Cineol, üblicherweise in einer peroralen Darreichungsform, vorzugsweise in Form von Kapseln, eingesetzt. Erfindungsgemäß besonders bevorzugt ist die Applikation des Cineols, insbesondere des 1,8-Cineols, in Form einer peroralen, magensaftresistenten, aber dünndarmlöslichen Darreichung, insbesondere Kapsel. Ganz besonders bevorzugt sind perorale, magensaftresistente, aber dünndarmlösliche Darreichungsformen, insbesondere Kapseln, welche Cineol, insbesondere 1,8-Cineol, als Reinstoff enthalten. Derartige Produkte sind im Handel erhältlich bzw. verfügbar (z. B. Soledum^{®}-Kapseln bzw. Soledum^{®} forte-Kapseln, vertrieben von der Cassella-med GmbH & Co. KG bzw. von der Maria Clementine Martin Klosterfrau Vertriebsgesellschaft mbH, jeweils Köln, Deutschland).

Im Allgemeinen ist es im Rahmen der vorliegenden Erfindung vorgesehen, dass die perorale Darreichungsform, insbesondere Kapsel, magensaftresistent, aber dünndarmlöslich ausgebildet ist. Hierdurch wird ein besonders optimales Freisetzungsprofil erreicht, da der Wirkstoff ziel- und zweckgerichtet erst im Darm freigesetzt und hiervon ausgehend optimal vom Körper aufgenommen wird.

Die im Rahmen dieser Erfindung verwendeten Definitionen für die Begriffe "magensaftresistent" bzw. "dünndarmlöslich" sowie die diesbezüglichen Testmethoden sind in European Pharmacopoeia 7.0, 04/2010: 1502, Seiten 707 bis 709, Stichwort "Capsules", Unterkapitel "Gastro-Resistant Capsules*"* sowie European Pharmacopoeia 7.1, 04/2011: 20901, Seiten 3331 und 3332, Stichwort "2.9.1 Disintegration of Tablets and Capsules*"* wiedergegeben.

Unter dem Begriff "magensaftresistent" ist dabei im Rahmen der vorliegenden Erfindung insbesondere zu verstehen, dass die Kapseln mindestens zwei Stunden lang in 0,1 N Salzsäure, welche auf Temperaturen von 35 bis 39 °C erwärmt wird, unter ständiger Durchmischung aufbewahrt, insbesondere gerührt werden kann, ohne dass die Kapseln Anzeichen einer Zersetzung bzw. Risse oder andere Beschädigungen aufweisen.

Unter dem Begriff "dünndarmlöslich" ist im Rahmen der vorliegenden Erfindung hingegen insbesondere zu verstehen, dass die Kapseln in einer wässrigen Phosphatpuffer-Lösung, welche auf einen pH-Wert von etwa 6,8 eingestellt, unter Rühren bei Temperaturen im Bereich von 35 bis 39 °C innerhalb einer Stunde soweit zersetzt werden, dass die Wirksubstanz freigesetzt wird.

Im Rahmen der vorliegenden Erfindung ist es insbesondere vorgesehen, dass das Cineol, vorzugsweise 1,8-Cineol, in pharmazeutisch wirksamen bzw. therapeutisch wirksamen Mengen verabreicht wird und/oder dass das Cineol, vorzugsweise 1,8-Cineol, zur Verabreichung in pharmazeutisch wirksamen bzw. therapeutisch wirksamen Mengen hergerichtet ist.

Insbesondere kann es im Rahmen der vorliegenden Erfindung gemäß diesem Erfindungsaspekt nach einer besonderen Ausführungsform vorgesehen sein, dass das Cineol, vorzugsweise 1,8-Cineol, mit einer Tagesdosis im Bereich von 1 mg/diem bis 5.000 mg/diem, insbesondere im Bereich von 2 mg/diem bis 3.000 mg/diem, vorzugsweise im Bereich von 5 mg/diem bis 2.500 mg/diem, bevorzugt im Bereich von 10 mg/diem bis 2.000 mg/diem, besonders bevorzugt im Bereich von 50 mg/diem bis 1.500 mg/diem, ganz besonders bevorzugt im Bereich von 100 mg/diem bis 1.200 mg/diem, weiter bevorzugt im Bereich von 200 mg/diem bis 1.000 mg/diem, verabreicht wird und/oder dass das Cineol, vorzugsweise 1,8-Cineol, zur Verabreichung mit einer Tagesdosis im Bereich von 1 mg/diem bis 5.000 mg/diem, insbesondere im Bereich von 2 mg/diem bis 3.000 mg/diem, vorzugsweise im Bereich von 5 mg/diem bis 2.500 mg/diem, bevorzugt im Bereich von 10 mg/diem bis 2.000 mg/diem, besonders bevorzugt im Bereich von 50 mg/diem bis 1.500 mg/diem, ganz besonders bevorzugt im Bereich von 100 mg/diem bis 1.200 mg/diem, weiter bevorzugt im Bereich von 200 mg/diem bis 1.000 mg/diem, hergerichtet ist.

Gemäß der vorliegenden Erfindung ist es nach diesem Erfindungsaspekt vorgesehen, dass das Cineol, vorzugsweise 1,8-Cineol, als Reinstoff vorliegt bzw. verabreicht wird. Insbesondere kann dabei das Cineol, vorzugsweise 1,8-Cineol, mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, vorzugsweise 1,8-Cineol, vorliegen bzw. verabreicht werden.

In diesem Zusammenhang ist es erfindungsgemäß vorgesehen, dass das Cineol, vorzugsweise 1,8-Cineol, frei von anderen Terpenen ist bzw. dass das Cineol, vorzugsweise 1,8-Cineol, keine anderen Terpene enthält.

Wie vorstehend geschildert, ist es erfindungsgemäß vorgesehen, dass die monoterpenbasierte Wirksubstanz (d. h. Cineol, vorzugsweise 1,8-Cineol) in Form einer Reinsubstanz, d. h. als alleiniger bzw. technisch isolierter/aufgereinigter Wirkstoff, eingesetzt wird. Auf diese Weise kann beispielsweise eine immer gleichbleibende Dosierung bzw. ein immer gleicher Gehalt an Wirksubstanz in der erfindungsgemäß vorgesehenen Darreichungsform gewährleistet werden. Auch sind auf diese Weise hohe Wirkstoffdosen bzw. -mengen auch am Wirkort realisierbar. Zudem wird hierdurch das Wirkspektrum weiterführend fokussiert und Nebenwirkungen weiterführend verringert.

Vor allem besitzt - wie zuvor erläutert - Cineol, insbesondere 1,8-Cineol, in Reinform bzw. als Reinstoff ein besonders effizientes steroidartiges Wirkungspotential, da es in Reinform bzw. als Reinstoff nämlich eine besonders effiziente Hemmung von Entzündungsmediatoren bzw. der Leukotrien- und Zytokinproduktion bewirkt. Ätherische Mischöle, welche Cineol als eine von vielen Wirkkomponenten enthalten (wie z. B. Eukalyptusöl etc.) dagegen stimulieren die Prostaglandinproduktion und zeigen nur eine gegenüber reinem Cineol signifikant verminderte Hemmung der Leukotrien- und Zytokinproduktion, da die Mischöle auch solche Substanzen enthalten, welche die Zellaktivität und die Mediatorproduktion stimulieren können und daher nicht entzündungshemmend wirken, sondern vielmehr Unverträglichkeitsreaktionen verursachen können (d. h. derartige ätherische Mischöle bzw. Ölgemische können folglich im Allgemeinen die Zellaktivität erhöhen und sogar eine Entzündungsmediatorproduktion induzieren). Im Unterschied hierzu jedoch bewirkt isoliertes bzw. reines Cineol, insbesondere 1,8-Cineol, ausschließlich eine signifikante Hemmung der Entzündungsmediatorproduktion.

Gemäß einer wiederum weiteren besonderen Ausführungsform gemäß diesem Erfindungsaspekt kann es erfindungsgemäß vorgesehen sein, dass das Cineol, vorzugsweise 1,8-Cineol, zusammen mit mindestens einem physiologisch unbedenklichen Träger (Exzipienten), vorliegt und/oder verabreicht wird. Insbesondere kann es dabei vorgesehen sein, dass der physiologisch unbedenkliche Träger (Exzipient) mit 1,8-Cineol mischbar und/oder hierin löslich ist. Weiterhin kann es insbesondere dabei vorgesehen sein, dass der physiologisch unbedenkliche Träger (Exzipient) bei 20 °C und bei Atmosphärendruck im flüssigen oder festen, vorzugsweise flüssigen, Aggregatzustand vorliegt.

Wiederum weiterhin kann es in diesem Zusammenhang vorgesehen sein, dass der physiologisch unbedenkliche Träger (Exzipient) ausgewählt sein kann aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

In diesem Zusammenhang ist es erfindungsgemäß vorgesehen, dass das Cineol, vorzugsweise 1,8-Cineol, als Reinstoff und in Abwesenheit von anderen (weiteren) Terpenen vorliegt bzw. verabreicht wird.

In diesem Zusammenhang kann es gleichermaßen vorgesehen sein, dass das Cineol, vorzugsweise 1,8-Cineol, als Reinstoff bzw. in Abwesenheit von anderen (weiteren) Terpenen, zusammen mit mindestens einem physiologisch unbedenklichen Träger (Exzipienten) vorliegt bzw. verabreicht wird.

Gemäß einer besonderen Ausführungsform enthält somit - wie zuvor geschildert - die erfindungsgemäß vorgesehene Darreichungsform das Monoterpen und den Wirkstoff Cineol, insbesondere 1,8-Cineol, zusammen mit mindestens einem mit der Wirksubstanz mischbaren und/oder hierin löslichen, insbesondere bei 20 °C und Atmosphärendruck flüssigen, physiologisch unbedenklichen Träger (Exzipienten). Der Träger bzw. Exzipient sollte selbst pharmakologisch nicht wirksam sein, aber mit der Wirksubstanz eine vorzugsweise homogene Mischung oder Lösung bilden.

Gemäß einer erfindungsgemäß bevorzugten Ausführungsform kann also dabei, wie zuvor erläutert, der Träger bzw. Exzipient ausgewählt sein aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Als Träger bzw. Exzipient bevorzugt eingesetzt werden können insbesondere Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den sogenannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe "*tri*" auf drei Acyl-Säurereste, die mit Glycerin verestert sind, verweist.

Speziell mittelkettige Triglyceride, wie sie erfindungsgemäß bevorzugt als Träger bzw. Exzipient für die monoterpenhaltige Wirksubstanz zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂-Ketten). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, welches aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur., 6. Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*"*).

Erfindungsgemäß bevorzugt ist es, wenn der Träger bzw. Exzipient in einem Wirksubstanz/Träger-Mengenverhältnis im Bereich von 1.000 : 1 bis 1 : 1.000, insbesondere 100 : 1 bis 1 : 100, vorzugsweise 50 : 1 bis 1 : 50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird. Durch die Mischung der Wirksubstanz (d. h. Cineol, insbesondere 1,8-Cineol) mit dem Exzipienten werden eine deutlich verbesserte Kompatibilität der Wirksubstanz mit den weiteren Inhaltsstoffen der Darreichungsform und eine verbesserte Stabilität, insbesondere Lagerstabilität, erreicht.

Gemäß einer gleichermaßen weiteren besonderen Ausführungsform der vorliegenden Erfindung kann es vorgesehen sein, dass das Cineol, vorzugsweise 1,8-Cineol, in bzw. in Form einer Zusammensetzung, insbesondere pharmazeutischen Zusammensetzung, insbesondere zusammen mit mindestens einem pharmazeutisch verträglichen und/oder physiologisch unbedenklichen Exzipienten (Träger), vorliegt und/oder verabreicht wird.

**In** diesem Zusammenhang kann es insbesondere vorgesehen sein, dass die Zusammensetzung das Cineol, vorzugsweise 1,8-Cineol, als alleinigen Wirkstoff, insbesondere als alleinigen pharmazeutischen Wirkstoff, bzw. als Einzelwirkstoff enthält.

Weiterhin ist es dabei vorgesehen, dass die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, vorzugsweise 1,8-Cineol, und frei von anderen Terpenen.

Gleichermaßen ist es dabei auch vorgesehen, dass die Zusammensetzung das Cineol, vorzugsweise 1,8-Cineol, frei von anderen Terpenen enthält und/oder dass die Zusammensetzung kein weiteres Terpen enthält und/oder dass die Zusammensetzung frei von anderen Terpenen als Cineol, vorzugsweise 1,8-Cineol, ist bzw. ausgebildet ist.

Weiterhin kann es dabei vorgesehen sein, dass die Zusammensetzung das Cineol, vorzugsweise 1,8-Cineol, in wirksamen, insbesondere pharmazeutisch und/oder therapeutisch wirksamen, Mengen enthält.

Ebenfalls kann es in diesem Kontext erfindungsgemäß vorgesehen sein, dass die Zusammensetzung das Cineol, vorzugsweise 1,8-Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

Schließlich kann es erfindungsgemäß in diesem Zusammenhang auch vorgesehen sein, dass der mindestens eine pharmazeutisch verträgliche und/oder physiologisch unbedenkliche Exzipient (Träger) mit 1,8-Cineol mischbar und/oder hierin löslich ist. Vorzugsweise kann der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegen. Ebenfalls bevorzugt kann der mindestens eine pharmazeutisch verträgliche und/oder physiologisch unbedenkliche Exzipient (Träger) ausgewählt sein aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Gemäß einer besonderen Ausführungsform der vorliegenden Erfindung kann das Cineol, vorzugsweise 1,8-Cineol, in liposomenumgebener und/oder liposomal verpackter Form vorliegt und/oder verabreicht werden. Hierdurch ist auch ein besonders gutes Freisetzungsprofil gewährleistet.

Gemäß einer weiteren besonderen Ausführungsform der vorliegenden Erfindung kann das Cineol, vorzugsweise 1,8-Cineol, in micellierter Form bzw. in Form einer micellaren Darreichungsform vorliegen und/oder verabreicht werden. Auch hierdurch kann ein besonders gutes Freisetzungsprofil gewährleistet werden.

Gemäß einer ersten erfindungsgemäßen Ausführungsform kann das Cineol, vorzugsweise 1,8-Cineol, als Einzelwirkstoff bzw. als Monopräparat vorliegen bzw. verabreicht werden bzw. zur Verabreichung hergerichtet sein. Diesbezüglich kann das Cineol, vorzugsweise 1,8-Cineol, ohne bzw. in Abwesenheit von weiteren, von Cineol, vorzugsweise 1,8-Cineol, verschiedenen Wirkstoffen vorliegen bzw. verabreicht werden.

Insbesondere kann das Cineol, vorzugsweise 1,8-Cineol, ohne und/oder in Abwesenheit von weiteren, von Cineol, vorzugsweise 1,8-Cineol, verschiedenen Wirkstoffen vorliegen bzw. verabreicht werden bzw. zur Verabreichung hergerichtet sein.

Erfindungsgemäß kann das Cineol, vorzugsweise 1,8-Cineol, ohne und/oder in Abwesenheit von und/oder frei von und/oder nicht in Kombination mit Vitamin D-Rezeptoragonisten (VDA), insbesondere Verbindungen der Vitamin D-Gruppe, vorliegen bzw. verabreicht werden bzw. zur Verabreichung hergerichtet sein.

Gemäß einer weiteren erfindungsgemäßen Ausführungsform kann es jedoch vorgesehen sein, dass das Cineol, vorzugsweise 1,8-Cineol, mit mindestens einem weiteren Wirkstoff bzw. als Co-Therapeutikum vorliegt bzw. verabreicht wird. In diesem Zusammenhang kann es insbesondere vorgesehen sein, dass der weitere Wirkstoff ausgewählt ist aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Gemäß einer weiterführend bevorzugten Ausführungsform kann es dabei insbesondere vorgesehen sein, dass der weitere Wirkstoff getrennt, insbesondere räumlich getrennt, von dem Cineol, vorzugsweise 1,8-Cineol, aber funktional zusammenhängend hiermit eingesetzt bzw. verabreicht wird, insbesondere in Form eines Kit (Kit-of-part).

Durch die gezielte Kombination oder gemeinsame Verabreichung von Cineol bzw. 1,8-Cineol mit mindestens einem weiteren Wirkstoff gelingt es im Rahmen der vorliegenden Erfindung insbesondere, die Wirkung bzw. Effizienz des weiteren Wirkstoffs signifikant, insbesondere in synergistischer Weise, zu steigern und dessen eingesetzte Dosismenge signifikant zu reduzieren, verbunden mit den damit einhergehenden Vorteilen (z. B. Vermeidung bzw. Reduzierung von Nebenwirkungen oder dergleichen).

Wie zuvor ausgeführt, kann es im Rahmen der vorliegenden Erfindung vorgesehen sein, dass das Cineol als Co-Therapeutikum bzw. als Co-Medikation im Rahmen einer Otitis-Basistherapie eingesetzt wird bzw. dass das Cineol, vorzugsweise 1,8-Cineol, als oder in Kombinationstherapie zu einer Otitis-Basistherapie eingesetzt wird. Wie zuvor angeführt, kann das erfindungsgemäß eingesetzte Cineol, vorzugsweise 1,8-Cineol, zu einer signifikanten, insbesondere synergistischen Wirksteigerung des oder der Basistherapeutika, wie zuvor angeführt, führen.

Die vorliegende Erfindung sowohl gemäß dem ersten Aspekt der vorliegenden Erfindung als auch gemäß sämtlichen übrigen Aspekten der vorliegenden Erfindung ist somit mit einer Vielzahl von Vorteilen und Besonderheiten verbunden, welche das erfindungsgemäße Therapiekonzept einzigartig und besonders, insbesondere hocheffizient, machen.

Für weitergehende Einzelheiten zu dem vorstehend beschriebenen ersten Erfindungsaspekt kann zudem Bezug genommen werden auf die Ausführungen zu den weiteren Erfindungsaspekten, wobei diese Ausführungen gleichermaßen für den vorliegenden ersten Erfindungsaspekt gelten.

Mit anderen Worten betrifft die vorliegende Offenbarung (nicht Teil der beanspruchten Erfindung) die Verwendung von Cineol, vorzugsweise 1,8-Cineol, zur prophylaktischen und/oder therapeutischen Behandlung von Otitis media mit begleitender (einhergehender) Otorrhoe, wobei das Cineol systemisch appliziert wird, wobei das Cineol als Reinstoff vorliegt und wobei das Cineol frei von anderen Terpenen ist; wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

In diesem Zusammenhang betrifft die vorliegende Offenbarung (nicht Teil der beanspruchten Erfindung) auch die Verwendung von Cineol, vorzugsweise 1,8-Cineol, zur Herstellung eines Arzneimittels oder Medikaments zur prophylaktischen und/oder therapeutischen Behandlung von Otitis media mit begleitender (einhergehender) Otorrhoe, wobei das Cineol systemisch appliziert wird, wobei das Cineol als Reinstoff vorliegt und wobei das Cineol frei von anderen Terpenen ist; wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

Gemäß diesen **Verwendungen** kann das Cineol, vorzugsweise 1,8-Cineol, insbesondere das Arzneimittel oder Medikament oder die Zusammensetzung, in pharmazeutisch wirksamen Mengen bzw. therapeutisch wirksamen Mengen verabreicht werden. Diesbezüglich kann das Cineol, vorzugsweise 1,8-Cineol, insbesondere das Arzneimittel oder Medikament oder die Zusammensetzung, zur Verabreichung in pharmazeutisch wirksamen bzw. therapeutisch wirksamen Mengen hergerichtet sein. Zudem wird das Cineol, vorzugsweise 1,8-Cineol, insbesondere das Arzneimittel oder Medikament oder die Zusammensetzung, systemisch, vorzugsweise peroral, appliziert.

Erforderlichenfalls kann gemäß einer wiederum weiteren Ausführungsform gemäß diesem Aspekt das Cineol, vorzugsweise 1,8-Cineol, insbesondere das Arzneimittel oder Medikament oder die Zusammensetzung, zusammen mit mindestens einem weiteren Wirkstoff angewendet bzw. appliziert werden. Diesbezüglich kann der weitere Wirkstoff ausgewählt sein aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Für weitergehende Einzelheiten zu diesen Verwendungen kann Bezug genommen werden auf die Ausführungen zu den weiteren Erfindungsaspekten, wobei diese Ausführungen gleichermaßen für die vorliegenden Verwendungen gelten.

Gleichermaßen Gegenstand der vorliegenden Offenbarung (nicht Teil der beanspruchten Erfindung) ist die Verwendung von Cineol, vorzugsweise 1,8-Cineol, als Ohrbehandlungsmittel zur prophylaktischen und/oder therapeutischen Behandlung von Otitis media begleitender (einhergehender) Otorrhoe, wobei das Cineol systemisch appliziert wird, wobei das Cineol als Reinstoff vorliegt und wobei das Cineol frei von anderen Terpenen ist; wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

Gemäß dieser Verwendung kann das Cineol, vorzugsweise 1,8-Cineol, insbesondere das Arzneimittel oder Medikament oder die Zusammensetzung oder das Ohrbehandlungsmittel, in pharmazeutisch wirksamen Mengen bzw. therapeutisch wirksamen Mengen verabreicht werden. Diesbezüglich kann das Cineol, vorzugsweise 1,8-Cineol, insbesondere das Arzneimittel oder Medikament oder die Zusammensetzung, zur Verabreichung in pharmazeutisch wirksamen bzw. therapeutisch wirksamen Mengen hergerichtet sein. Zudem wird das Cineol, vorzugsweise 1,8-Cineol, insbesondere das Arzneimittel oder Medikament oder die Zusammensetzung, systemisch, vorzugsweise peroral, appliziert.

Erforderlichenfalls kann gemäß einer wiederum weiteren Ausführungsform gemäß dieser Verwendung das Cineol, vorzugsweise 1,8-Cineol, insbesondere das Arzneimittel oder Medikament oder die Zusammensetzung oder das Ohrbehandlungsmittel, zusammen mit mindestens einem weiteren Wirkstoff angewendet bzw. appliziert werden. Diesbezüglich kann der weitere Wirkstoff ausgewählt sein aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Für weitergehende Einzelheiten zu dieser Verwendung kann Bezug genommen werden auf die Ausführungen zu den weiteren Erfindungsaspekten, wobei diese Ausführungen gleichermaßen für die vorliegende Verwendung gelten.

Ebenfalls Gegenstand der vorliegenden Erfindung - gemäß einem **weiteren** Aspekt der vorliegenden Erfindung - ist das erfindungsgemäße Arzneimittel oder Medikament, insbesondere Ohrbehandlungsmittel, zur Verwendung bei der prophylaktischen und/oder therapeutischen Behandlung von Otitis media mit begleitender (einhergehender) Otorrhoe,
wobei das Arzneimittel oder Medikament, insbesondere Antivirusmittel, Cineol, vorzugsweise 1,8-Cineol, zusammen mit mindestens einem pharmazeutisch verträglichen und/oder physiologisch unbedenklichen Exzipienten (Träger) enthält,
wobei das Arzneimittel oder Medikament systemisch appliziert wird,
wobei das Arzneimittel oder Medikament das Cineol als Reinstoff enthält und wobei das Arzneimittel oder Medikament frei von anderen Terpenen als Cineol ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

Auch im Rahmen dieses Erfindungsaspekts ist es insbesondere vorgesehen, dass der Wirkstoff bzw. das Cineol, vorzugsweise 1,8-Cineol, bzw. das Arzneimittel oder Medikament, insbesondere Ohrbehandlungsmittel, systemisch appliziert wird.

Erfindungsgemäß bevorzugt ist es, wenn das Arzneimittel oder Medikament das Cineol, vorzugsweise 1,8-Cineol, als alleinigen Wirkstoff, insbesondere als alleinigen pharmazeutischen Wirkstoff, bzw. als Einzelwirkstoff enthält.

Gemäß diesem Erfindungsaspekt enthält das erfindungsgemäße Arzneimittel oder Medikament das Cineol, vorzugsweise 1,8-Cineol, als Reinstoff, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, vorzugsweise 1,8-Cineol, und frei von anderen Terpenen.

Gemäß diesem Erfindungsaspekt ist es gleichermaßen sein, dass das Arzneimittel oder Medikament das Cineol, vorzugsweise 1,8-Cineol, frei von anderen Terpenen enthält und/oder dass das Arzneimittel oder Medikament kein weiteres Terpen enthält und/oder dass das Arzneimittel oder Medikament frei von anderen Terpenen als Cineol, vorzugsweise 1,8-Cineol, ist.

Gemäß einer wiederum weiteren besonderen Ausführungsform gemäß diesem Erfindungsaspekt kann es vorgesehen sein, dass das erfindungsgemäße das Arzneimittel oder Medikament das Cineol, vorzugsweise 1,8-Cineol, in wirksamen, insbesondere pharmazeutisch und/oder therapeutisch wirksamen, Mengen enthält.

Insbesondere kann es vorgesehen sein, dass das erfindungsgemäße Arzneimittel oder Medikament das Cineol, vorzugsweise 1,8-Cineol, bezogen auf das Arzneimittel oder Medikament, in relativen Mengen im Bereich von 0,0001 bis 80 Gew.-%, insbesondere 0,001 bis 75 Gew.-%, vorzugsweise 0,005 bis 70 Gew.- %, bevorzugt 0,01 bis 60 Gew.-%, besonders bevorzugt 0,05 bis 55 Gew.-%, ganz besonders bevorzugt 0,1 bis 50 Gew.-%, enthält.

Gemäß einer wiederum weiteren besonderen Ausführungsform gemäß diesem Erfindungsaspekt kann es weiterhin vorgesehen sein, dass der mindestens eine pharmazeutisch verträgliche bzw. physiologisch unbedenkliche Exzipient (Träger) mit 1,8-Cineol mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Erfindungsgemäß kann es insbesondere vorgesehen sein, dass das Arzneimittel oder Medikament keine weiteren, von Cineol, vorzugsweise 1,8-Cineol, verschiedenen Wirkstoffe enthält bzw. dass das Arzneimittel oder Medikament frei von anderen Wirkstoffen als Cineol, vorzugsweise 1,8-Cineol, ist bzw. dass das Arzneimittel oder Medikament nicht in Kombination mit weiteren, von Cineol, vorzugsweise 1,8-Cineol, verschiedenen Wirkstoffen vorliegt und/oder verabreicht wird und/oder zur Verabreichung hergerichtet ist.

Weiterhin kann es im Rahmen der vorliegenden Erfindung vorgesehen sein, dass das Arzneimittel oder Medikament keine Vitamin D-Rezeptoragonisten (VDA), insbesondere keine Verbindungen der Vitamin D-Gruppe, enthält bzw. dass das Arzneimittel oder Medikament frei von Vitamin D-Rezeptoragonisten (VDA), insbesondere frei von Verbindungen der Vitamin D-Gruppe, ist bzw. dass das Arzneimittel oder Medikament nicht in Kombination mit Vitamin D-Rezeptoragonisten (VDA), insbesondere nicht in Kombination mit Verbindungen der Vitamin D-Gruppe, vorliegt und/oder verabreicht wird und/oder zur Verabreichung hergerichtet ist.

Demgegenüber kann es auch im Rahmen dieses Erfindungsaspekts erforderlichenfalls vorgesehen sein, dass der Wirkstoff bzw. das Cineol, vorzugsweise 1,8-Cineol, bzw. das Arzneimittel oder Medikament, insbesondere Ohrbehandlungsmittel, zusammen mit mindestens einem weiteren Wirkstoff angewendet oder appliziert wird. Der weitere Wirkstoff kann insbesondere ausgewählt sein aus (i) entzündungshemmenden Wirkstoffen (Antiphlogistika), insbesondere nicht steroidalen Entzündungshemmern (NSAIDs) oder steroidalen Entzündungshemmern; (ii) Analgetika; (iii) Antipyretika; (iv) Antibiotika; sowie deren Kombinationen.

Gemäß diesem erfindungsgemäßen Aspekt kann das Cineol, vorzugsweise 1,8-Cineol, insbesondere das Arzneimittel oder Medikament, in pharmazeutisch wirksamen Mengen bzw. therapeutisch wirksamen Mengen verabreicht werden. Diesbezüglich kann das Cineol, vorzugsweise 1,8-Cineol, insbesondere das Arzneimittel oder Medikament oder die Zusammensetzung, zur Verabreichung in pharmazeutisch wirksamen bzw. therapeutisch wirksamen Mengen hergerichtet sein. Zudem kann das Cineol, vorzugsweise 1,8-Cineol, insbesondere das Arzneimittel oder Medikament oder die Zusammensetzung, systemisch, vorzugsweise peroral, appliziert werden.

Erforderlichenfalls kann gemäß einer wiederum weiteren Ausführungsform gemäß diesem Aspekt das Cineol, vorzugsweise 1,8-Cineol, insbesondere das Arzneimittel oder Medikament, zusammen mit mindestens einem weiteren Wirkstoff angewendet bzw. appliziert werden. Diesbezüglich kann der weitere Wirkstoff ausgewählt sein aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Für weitergehende Einzelheiten zu diesem Erfindungsaspekt kann auch Bezug genommen werden auf die weiteren Ausführungen zu den vorliegend beschriebenen Erfindungsaspekten, wobei diese Ausführungen gleichermaßen für den vorliegenden Erfindungsaspekt gelten.

Wiederum weiterer Gegenstand der vorliegenden Erfindung - gemäß einem **weiteren** Aspekt der vorliegenden Erfindung - ist zudem die erfindungsgemäße Zusammensetzung, insbesondere pharmazeutische Zusammensetzung, zur Verwendung bei der prophylaktischen und/oder therapeutischen Behandlung von Otitis media mit begleitender (einhergehender) Otorrhoe,
wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält,
wobei die Zusammensetzung systemisch appliziert wird,
wobei die Zusammensetzung das Cineol als Reinstoff enthält und wobei die Zusammensetzung frei von anderen Terpenen als Cineol ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

Auch im Rahmen dieses Erfindungsaspekts ist es insbesondere vorgesehen, dass die Zusammensetzung das Cineol als alleinigen Wirkstoff, insbesondere als alleinigen pharmazeutischen Wirkstoff, bzw. als Einzelwirkstoff enthält.

Erfindungsgemäß ist es vorgesehen, dass die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, vorzugsweise 1,8-Cineol, und vorzugsweise frei von anderen Terpenen.

Erfindungsgemäß enthält die Zusammensetzung das Cineol, vorzugsweise 1,8-Cineol, frei von anderen Terpenen. Diesbezüglich ist es der Fall, dass die Zusammensetzung kein weiteres Terpen enthält bzw. dass die Zusammensetzung frei von anderen Terpenen als Cineol, vorzugsweise 1,8-Cineol, ist. Insbesondere kann die Zusammensetzung das Cineol, vorzugsweise 1,8-Cineol, in wirksamen, insbesondere pharmazeutisch und/oder therapeutisch wirksamen, Mengen enthalten.

Diesbezüglich kann die Zusammensetzung das Cineol, vorzugsweise 1,8-Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthalten.

Erfindungsgemäß kann es gemäß dem vorliegenden Aspekt auch vorgesehen sein, dass der mindestens eine pharmazeutisch verträgliche und/oder physiologisch unbedenkliche Exzipient (Träger) mit 1,8-Cineol mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Gemäß diesem erfindungsgemäßen Aspekt kann das Cineol, vorzugsweise 1,8-Cineol, insbesondere das Arzneimittel oder Medikament oder die Zusammensetzung, in pharmazeutisch wirksamen Mengen bzw. therapeutisch wirksamen Mengen verabreicht werden. Diesbezüglich kann das Cineol, vorzugsweise 1,8-Cineol, insbesondere das Arzneimittel oder Medikament oder die Zusammensetzung, zur Verabreichung in pharmazeutisch wirksamen bzw. therapeutisch wirksamen Mengen hergerichtet sein. Zudem kann das Cineol, vorzugsweise 1,8-Cineol, insbesondere das Arzneimittel oder Medikament oder die Zusammensetzung, systemisch, vorzugsweise peroral, appliziert werden.

Erfindungsgemäß kann es insbesondere vorgehen sein, dass die Zusammensetzung keine weiteren, von Cineol, vorzugsweise 1,8-Cineol, verschiedenen Wirkstoffe enthält bzw. dass die Zusammensetzung frei von anderen Wirkstoffen als Cineol, vorzugsweise 1,8-Cineol, ist bzw. dass die Zusammensetzung nicht in Kombination mit weiteren, von Cineol, vorzugsweise 1,8-Cineol, verschiedenen Wirkstoffen vorliegt und/oder verabreicht wird und/oder zur Verabreichung hergerichtet ist.

Insbesondere kann es vorgesehen sein, dass die Zusammensetzung keine Vitamin D-Rezeptoragonisten (VDA), insbesondere keine Verbindungen der Vitamin D-Gruppe, enthält bzw. dass die Zusammensetzung frei von Vitamin D-Rezeptoragonisten (VDA), insbesondere frei von Verbindungen der Vitamin D-Gruppe, ist bzw. dass die Zusammensetzung nicht in Kombination mit Vitamin D-Rezeptoragonisten (VDA), insbesondere nicht in Kombination mit Verbindungen der Vitamin D-Gruppe, vorliegt und/oder verabreicht wird und/oder zur Verabreichung hergerichtet ist.

Erforderlichenfalls kann demgegenüber gemäß einer wiederum weiteren Ausführungsform gemäß diesem Aspekt das Cineol, vorzugsweise 1,8-Cineol, insbesondere das Arzneimittel oder Medikament oder die Zusammensetzung, zusammen mit mindestens einem weiteren Wirkstoff angewendet bzw. appliziert werden. Diesbezüglich kann der weitere Wirkstoff ausgewählt sein aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Für weitergehende Einzelheiten zu diesem Erfindungsaspekt kann Bezug genommen werden auf die weiteren Ausführungen zu den vorliegend beschriebenen Erfindungsaspekten, wobei diese Ausführungen gleichermaßen für den vorliegend beschriebenen Erfindungsaspekt gelten.

Schließlich ist Gegenstand der vorliegenden Erfindung - gemäß einem **weiteren** Aspekt der vorliegenden Erfindung - gleichermaßen eine pharmazeutische Kombination zur Verwendung bei der prophylaktischen und/oder therapeutischen Behandlung von Otitis media mit begleitender (einhergehender) Otorrhoe,
wobei die pharmazeutische Kombination mindestens die nachfolgenden Komponenten (A) und (B) umfasst:
(A) Cineol, vorzugsweise 1,8-Cineol,
   wobei das Cineol systemisch appliziert wird,
   wobei das Cineol als Reinstoff vorliegt und wobei das Cineol frei von anderen Terpenen ist,
   wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird; sowie
(B) mindestens einen weiteren Wirkstoff, ausgewählt aus (i) entzündungshemmenden Wirkstoffen (Antiphlogistika), insbesondere nichtsteroidalen Entzündungshemmern (NSAIDs) oder steroidalen Entzündungshemmern; (ii) Analgetika; (iii) Antipyretika; (iv) Antibiotika; sowie deren Kombinationen.

Auch gemäß diesem Aspekt der vorliegenden Erfindung ist es vorgesehen, dass der Wirkstoff der Komponente (A) bzw. das Cineol, vorzugsweise 1,8-Cineol, systemisch, vorzugsweise peroral, appliziert wird.

Auch der weitere Wirkstoff (B) kann insbesondere gleichermaßen systemisch angewendet bzw. appliziert werden.

Gemäß einer besonderen Ausführungsform dieses Erfindungsaspekts kann es insbesondere vorgesehen sein, dass die Komponenten (A) und (B) getrennt voneinander, insbesondere räumlich getrennt voneinander, aber funktional zusammenhängend und/oder funktional einander zugeordnet vorliegen und/oder verabreicht werden.

Weiterhin kann es gemäß einer wiederum besonderen Ausführungsform dieses Erfindungsaspekts insbesondere vorgesehen sein, dass die pharmazeutische Kombination in Form eines Kit (Kit-of-parts), insbesondere als Kit der Komponenten (A) und (B), vorliegt.

Insbesondere können die Komponenten (A) und (B) als Kit (Kit-of-parts) vorliegen bzw. hergerichtet sein bzw. verabreicht werden.

Als Kit bzw. Kit-of-parts wird im Rahmen der vorliegenden Erfindung insbesondere eine Einheit bzw. Anordnung bzw. Zusammenstellung bzw. Kombination der beiden Komponenten (A) und (B) verstanden, bei welcher die beiden Komponenten (A) und (B) zwar getrennt und/oder separat, insbesondere räumlich getrennt und/oder räumlich separiert, vorliegen, aber als funktional zusammengehörende bzw. als funktional einander zugeordnete Komponenten vorgesehen sind bzw. verabreicht werden.

Für weitergehende Einzelheiten zu diesem Erfindungsaspekt kann Bezug genommen werden auf die Ausführungen zu den weiteren Erfindungsaspekten, wobei diese Ausführung gleichermaßen für den vorliegenden Erfindungsaspekt gelten.

Weitere vorteilhafte Eigenschaften, Aspekte und Merkmale der vorliegenden Erfindung ergeben sich aus der folgenden Beschreibung von in den Figuren dargestellten Ausführungsbeispielen, wie sie nachfolgend noch im Detail angeführt sind. Es zeigt:

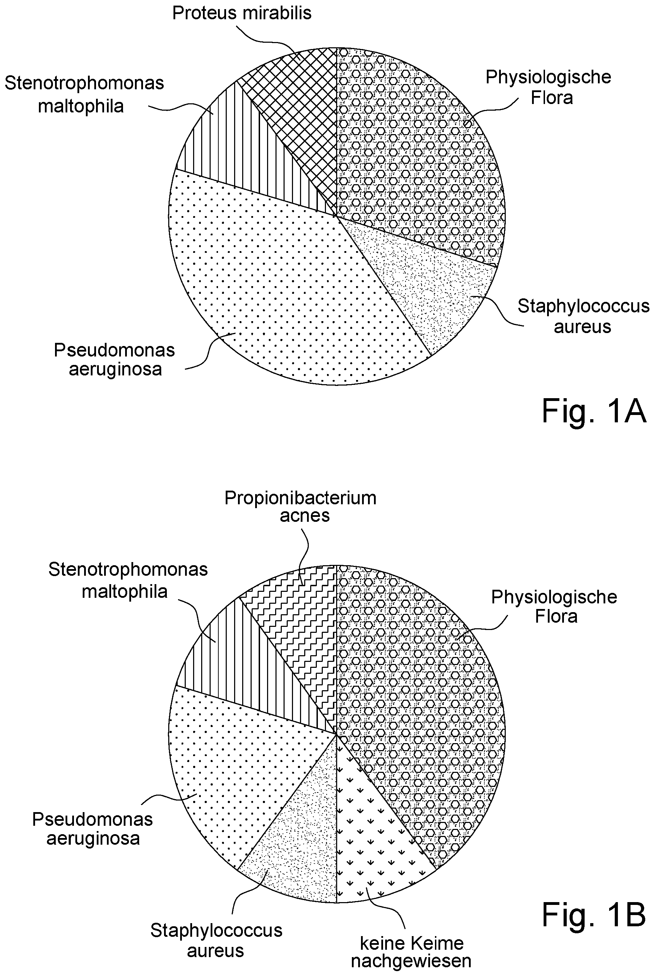

- Fig. 1A/B: eine jeweilige grafische Darstellung in Form von Kreisdiagrammen zur bakteriellen Besiedlung des Gehörgangs von Patienten mit Otitis media (n = 9; ein Patient mit Untersuchung beider Ohren, d.h. insgesamt zehn untersuchte Ohren) vor Verabreichung (Fig. 1A) und nach Verabreichung (Fig. 1B) von dreimal täglich 200 mg 1,8 Cineol (Verabreichungszeitraum: 14 Tage, perorale Verabreichung); die Untersuchungen zeigen deutliche Verschiebungen hin zu einer vermehrt physiologischen Besiedlung des Ohres, einhergehend mit einer verminderten Besiedlung durch pathogene Keime, wobei auch für *Pseudomonas aeroginosa* ein besonders deutlicher Rückgang festzustellen ist;

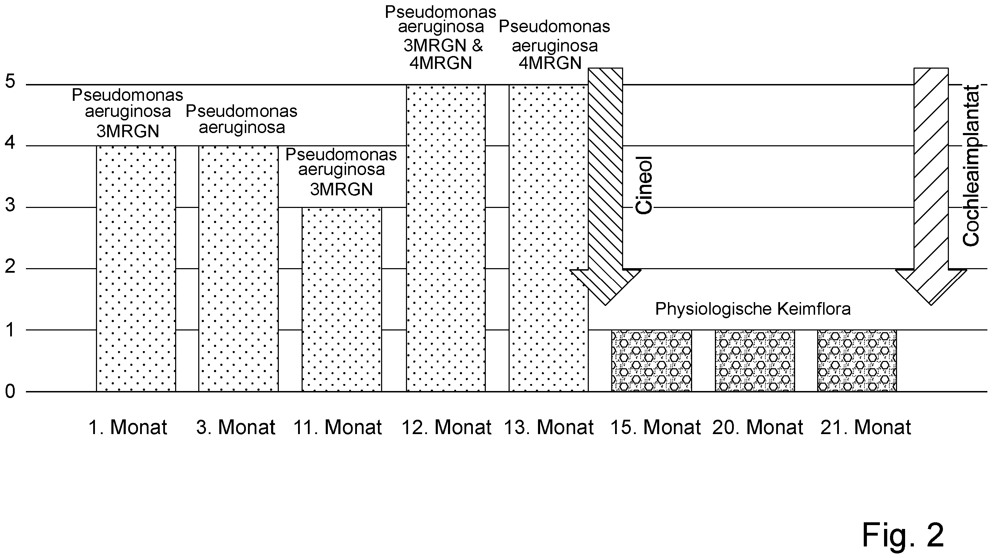

- Fig. 2: eine graphische Darstellung in Form eines Balkendiagramms mit der exemplarischen Auswertung der bakteriellen Besiedlung bei einem Patienten (mit chronischer Otitis media mesotympanalis mit chronischer Otorrhoe, perorale Verabreichung) im zeitlichen Verlauf vor und nach Verabreichung von dreimal täglich 200 mg 1,8-Cineol (Verabreichungszeitraum: 14 Tage); Testung auf Erreger und Resistenzen in der Routinediagnostik und Darstellung einer Verschiebung der mikrobiellen Besiedlung anhand des Befundes mittels der Skala (dargestellt auf der y-Achse) 5 = massenhaft, 4 = reichlich, 3 = mäßig, 2 = vereinzelt, 1 = physiologische Keimflora, 0 = keine Keime nachgewiesen;

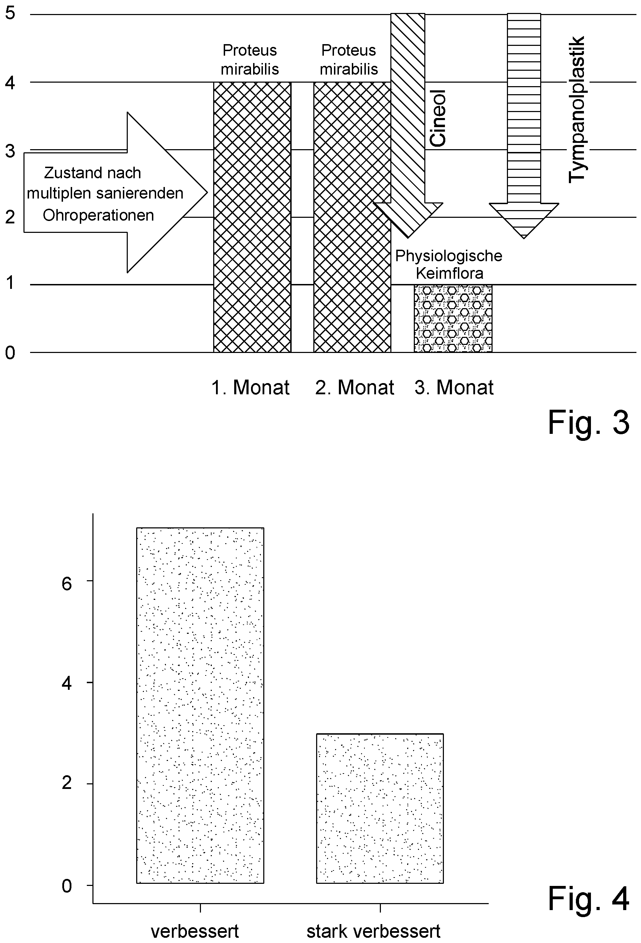

- Fig. 3: eine graphische Darstellung in Form eines Balkendiagramms mit der exemplarischen Darstellung des zeitlichen Verlaufs der Entwicklung bzw. des Zustands der Keimflora im Gehörgang eines Patienten (mit chronisch-granulierender Otitis media und Cholesteatom sowie foetider Otorrhoe unter Besiedelung durch *Proteus mirabilis*) vor und nach Behandlung mit 1,8-Cineol unter Auswertung mikrobieller Abstriche aus dem Gehörgang des Patienten vor und nach der Einnahme von dreimal täglich 200 mg 1,8-Cineol (Verabreichungszeitraum: 14 Tage, perorale Verabreichung); Testung auf Erreger und Resistenzen in der Routinediagnostik (Darstellung einer Verschiebung der mikrobiellen Besiedlung anhand des Befunds mittels der Skala (y-Achse) 5 = massenhaft, 4 = reichlich, 3 = mäßig, 2 = vereinzelt, 1 = physiologische Keimflora, 0 = keine Keime nachgewiesen);
- Fig. 4: eine schematische Darstellung in Form eines Balkendiagramms mit der Verdeutlichung der Verbesserung des klinischen Krankheitsbildes bei Patienten mit Otitis media im Hinblick auf das Vorliegen von Otorrhoe ("feuchtes Ohr") nach Behandlung mit dreimal täglich 200 mg 1,8-Cineol (Verabreichungszeitraum: 14 Tage, perorale Verabreichung); Auswertung auf Basis eines Patientenfragebogens an neun Patienten (bei einem Patienten wurden beide Ohren herangezogen und bei den weiteren Patienten jeweils ein Ohr); das Balkengram bezieht sich auf die Situation nach Behandlung mit 1,8-Cineol, wonach in sieben Fällen eine verbesserte Situation (linker Balken) und in drei Fällen eine stark verbesserte Situation (rechter Balken) in Bezug auf die Schwere der Otorrhoe angegeben wurde;

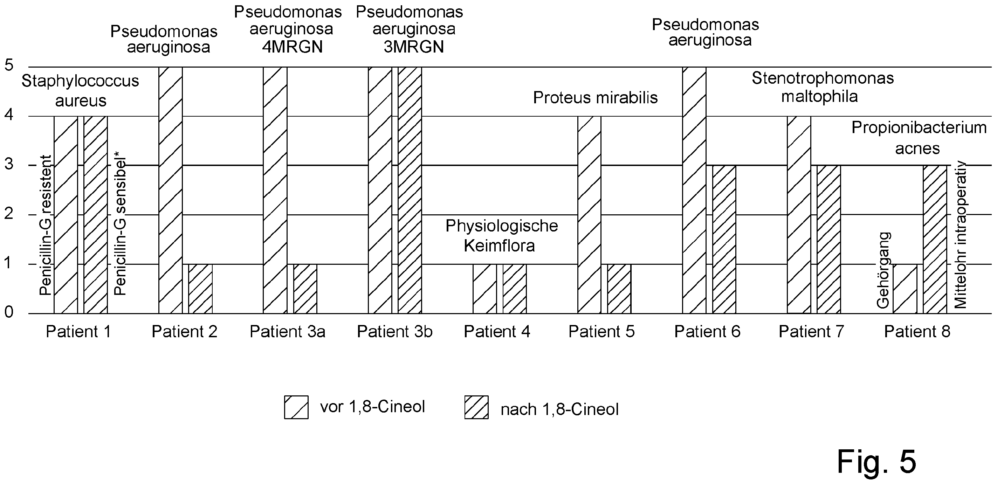

- Fig. 5: eine graphische Darstellung in Form eines Balkendiagramms zur Entwicklung der Besiedlung des Ohres mit Keimen auf Basis mikrobieller Abstriche aus dem Gehörgang von acht Patienten (ein Patient mit zwei untersuchen Ohren, vgl. Patient 3a bzw. 3b) mit chronischer Otitis media mesotympanalis bzw. Otitis media epitympanalis vor und nach Verabreichung von dreimal täglich 200 mg 1,8-Cineol (Verabreichungszeitraum: 14 Tage perorale Verabreichung); Testung auf Erreger und Resistenzen in der Routinediagnostik und Darstellung einer Verschiebung der mikrobiellen Besiedlung anhand des Befundes mittels der Skala (y-Achse) 5 = massenhaft, 4 = reichlich, 3 = mäßig, 2 = vereinzelt, 1 = physiologische Keimflora, 0 = keine Keime;

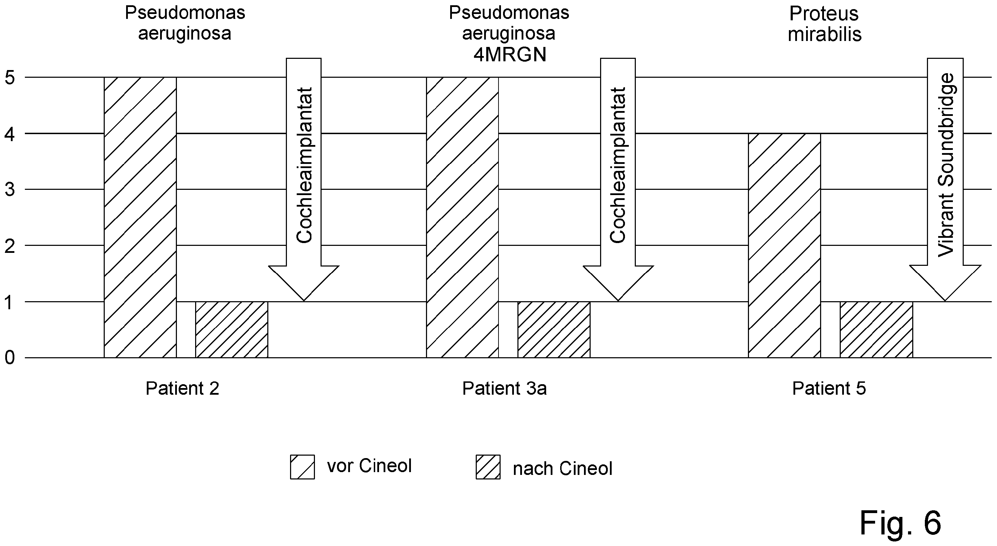

- Fig. 6: eine graphische Darstellung in Form eines Balkendiagramms mit einer exemplarischen Veranschaulichung der bakteriellen Besiedlung vor und nach Verabreichung von dreimal täglich 200 mg 1,8-Cineol unter Auswertung mikrobieller Abstriche aus dem Gehörgang bei drei Patienten vor und nach der oralen Verabreichung von dreimal täglich 200 mg 1,8-Cineol (Verabreichungszeitraum: 14 Tage, perorale Verabreichung); auch bei diesen Patienten konnte nach Verabreichung von 1,8-Cineol eine Reduktion der Entzündung mit Otorrhoe beobachtet werden und ein Implantat zur Verbesserung des Hörens und damit der Lebensqualität eingesetzt werden; Testung auf Erreger und Resistenzen in der Routinediagnostik und Darstellung an der Verschiebung der mikrobiellen Besiedelung anhand des ärztlichen Befundes mittels der Skala (y-Achse) 5 = massenhaft, 4 = reichlich, 3 = mäßig, 2 = vereinzelt, 1 = physiologische Keimflora, 0 = keine Keime.

Für weitergehende Einzelheiten zu den in den Figurendarstellungen dargestellten Ausführungsformen und Ausführungsbeispielen kann zur Vermeidung unnötiger Wiederholungen auch auf die obigen Ausführungen sowie auf die nachfolgenden ergänzenden Ausführungen zu den Ausführungsbeispielen verwiesen werden, welche in Bezug auf die Figurendarstellungen entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE:

Die nachfolgenden Ausführungsbeispiele zeigen den therapeutischen Nutzen von Cineol, insbesondere 1,8-Cineol, bei der Behandlung von Otitiden mit der vorliegend aufgezeigten Verringerung von pathogenen Keimen in den Ohren der von den Erkrankungen betroffenen Patienten.

Bei der nachfolgend beschriebenen Behandlung von Patienten kommt systemisches 1,8-Cineol in Reinform (zusammen mit einem Exzipienten auf Basis von mittelkettigen Triglyceriden (= *Medium Chain Triglycerides* bzw. MCT), d. h. Triglyceriden von C₆-C₁₂-Fettsäuren) als magensaftresistente dünndarmlösliche Kapseln zum Einsatz (nämlich das Handelspräparat Soledum^{®}-Kapseln bzw. Soledum^{®} forte-Kapseln, vertrieben von der Cassella-med GmbH & Co. KG bzw. von der Maria Clementine Martin Klosterfrau Vertriebsgesellschaft mbH, jeweils Köln, Deutschland).

### Beispiel 1:

### Bakterielle Besiedelung bei Otitiden

Im Rahmen einer Pilotstudie wurde die pathogene Keimbesiedlung von Patienten mit Otitiden untersucht. Hierzu wurden mikrobielle Abstriche aus dem Gehörgang von 22 Patienten mit chronischer Otitis media mesotympanalis bzw. epitympanalis entnommen und auf Erreger und Resistenzen in der Routinediagnostik getestet. Hierzu konnte bei den von der chronischen Otitis betroffenen Patienten eine Besiedlung mit pathogenen Keimen festgestellt werden. So traten bei Patienten mit chronischer Otitis media mesotympanalis neben der physiologischen Keimflora auch pathogene Keime in Form von *Pseudomonas aeroginosa* und *stutzeri* sowie *Proteus mirabilis* sowie *Staphylococcus aureus* auf (wobei bei einem einzigen Patienten konnte kein Keimnachweis erbracht werden). Bei Patienten mit insbesondere chronischer Otitis media epitympanalis traten neben der physiologischen Keimflora insbesondere pathogene Keime in Form von *Staphylococcus aureus* und *Stenotrophomonas maltophila* auf (wobei bei lediglich drei Patienten kein Keimnachweis erbracht werden konnte). Ein Patient wies sowohl *Enterococcus faecalis* als auch *Staphylococcus aureus* auf. Ein weiterer Patient wies *Aspergillus spp.* und *Candida parapsilosis* auf. Die obigen Ausführungen zeigen, dass bei Patienten mit Otitis media in entsprechender Weise pathogene Keime im Zusammenhang mit der Erkrankung auftreten.

### Beispiel 2:

### 1,8-Cineol wirkt pathogenen Keimen des Mittelohrs entgegen

Untersuchungen der bakteriellen Besiedlung aus dem Gehörgang von Otitis media Patienten (vgl. Fig. 1A sowie Fig. 1B; n = 9; ein Patient mit Untersuchung beider Ohren, d.h. insgesamt 10 untersuchte Ohren, Patienten mit insbesondere chronischer Otitis media mesotympanalis bzw. epitympanalis) zeigen durch Behandlung mit 1,8-Cineol deutliche Verschiebungen hin zu einer vermehrt physiologischen Ohrbesiedlung, einhergehend mit einer verminderten Besiedlung durch pathogene Keime, wobei auch für *Pseudomonas aeroginosa* ein besonders deutlicher Rückgang festzustellen ist, wobei zudem auch für die physiologische Keimflora eine deutliche Zunahme zu beobachten ist. Hierzu kann auch auf Fig. 1A sowie Fig. 1B verwiesen werden.

### Beispiel 3:

### Pathogene Keime bei chronischer Otitis media vor und nach Therapie mit 1,8-Cineol; Ermöglichung therapeutischer Operationen am Ohr (Fallbeispiel I)

Der erfindungsgemäß überraschend gefundene überaus positive Einfluss von 1,8-Cineol auf das Entzündungsgeschehen bzw. die Keimbesiedlung im Ohr von Patienten mit Otitiden, insbesondere auch im Hinblick auf eine deutliche Verringerung von Otorrhoe und eine Verringerung pathogener Keime im Ohr, ermöglichte im Rahmen der therapeutischen Anwendungen therapeutische Operationen am Ohr eines Patienten (Fig. 2, vgl. Patient 3 gemäß Fig. 5), welche vor Anwendung von 1,8-Cineol zum Teil nicht für möglich gehalten wurden bzw. nicht durchführbar waren. Hierzu kann auf Fig. 2 verwiesen werden, welche exemplarisch den diesbezüglichen Verlauf bei dem zugrundeliegenden Patienten (mit chronischer Otitis media mesotympanalis und chronischer Otorrhoe mit Besiedelung durch einen multiresistenten *Pseudomonas aeroginosa-Keim)* vor und nach Einnahme von 1,8-Cineol aufzeigt. Bei dem in Fig. 2 exemplarisch aufgezeigten Patienten konnte bei zuvor antibiotika- und therapieresistenten pathogenen Keimen nach Verabreichung von 1,8-Cineol bei trockenem Ohr und physiologischer Keimflora ein Cochleaimplantat implantiert werden. Die ist umso bemerkenswerter, als der Patient vor Behandlung eine starke Besiedlung mit einem multiresistenten *Pseudomonas aeroginosa-Keim* (in Fig. 2 mit MRGN (multiresistenter grammnegativer Erreger) bezeichnet) mit Resistenz gegenüber drei Antibiotikagruppen (3MRGN) bzw. sogar gegenüber vier Antibiotikagruppen (4MRGN) aufwies.

### Beispiel 4:

### Pathogene Keime bei chronischer Otitis media vor und nach Therapie mit 1,8-Cineol; Ermöglichung therapeutischer Operationen (Fallbeispiel II)

Weiterhin zeigt Fig. 3 exemplarisch den Verlauf für einen weiteren Patienten (mit chronisch-granulierender Otitis media und Cholesteatom sowie foetider Otorrhoe unter Besiedelung durch *Proteus mirabilis*) (Fig. 3, vgl. Patient 5 gemäß Fig. 5). Der Patient stellte sich anamnestisch mit Zustand nach multiplen sanierenden Ohroperationen (seit mehr als zehn Jahren) bei rezidivierendem Cholesteatom vor. Nach Verabreichung von 1,8-Cineol konnte bei physiologischer Keimflora nachfolgend eine Tympanoplastik (Typ III) mit Vibrant-Soundbridge-Implantation erfolgen, wobei die Operationen unter antibiotischer Abschirmung erfolgten.

### Beispiel 5:

### Auswertung von Patientenfragebögen: 1,8-Cineol verbessert das klinische Krankheitsbild und die Lebensqualität

Im Rahmen der therapeutischen Behandlung wurden Patienten mit 1,8-Cineol gemäß den Ausführungen zu Fig. 4 behandelt, wobei eine Auswertung anhand von Patientenfragebögen vorgenommen worden ist, und zwar auf Basis von neun Patienten (bei einem Patienten wurden beide Ohren herangezogen und bei den weiteren Patienten jeweils ein Ohr). Der Patientenfragebogen basiert dabei auf Scoring-Systemen bzw. Scoring-Klassifizierungen, anhand derer seitens der Patienten eine Bewertung hinsichtlich der jeweils zugrundeliegenden Fragen bzw. Aspekten vorgenommen wurde.

Das Patientenkollektiv setzte sich dabei aus Patienten im Alter von 27 Jahren bis 75 Jahren (30 % weiblich und 70 % männlich) zusammen. Vor Behandlung lag In sieben Fällen eine chronische Otitis media mesotympanalis, in einem Fall eine Trommelfellperforation, in einem weiteren Fall eine chronische Tubenbelüftungsstörung und in einem wiederum weiteren Fall eine chronische Otitis media mesotympanalis mit Mastoiditis vor, wobei in vier Fällen das rechte Ohr und in sechs Fällen das linke Ohr betroffen war. Im Hinblick auf die zugrundeliegenden Erkrankungen lag in einem Fall zudem eine sehr starke und in sechs Fällen eine starke Hörminderung vor. In einem weiteren Fall lag eine mittelgradige Hörbehinderung vor, und in zwei Fällen konnte eine geringfügige Hörminderung festgestellt werden.

Wie in Fig. 4 dargestellt, wurde anhand der Patientenfragebögen insbesondere eine signifikante Verbesserung hinsichtlich der Otorrhoe ("feuchtes Ohr") festgestellt werden (vor Therapie wurde in 20 % der Fälle ein gelegentliches Ohrenlaufen und für 80 % der Fälle ein ständiges ("immer") Ohrenlaufen angeführt). So wurde in 70 % der Fälle angeführt, dass das Ohrenlaufen nach Durchführung der Therapie verbessert ist. In 30 % der Fälle wurde sogar eine starke Verbesserung nach Therapie angeführt. Insbesondere hinsichtlich der starken Beeinträchtigung der Patienten durch ein "feuchtes Ohr" bzw. eine Otorrhoe konnte somit eine signifikante Verbesserung festgestellt werden.

Weiterhin konnten anhand der Patientenfragebögen auch positive Effekte der Therapie mit 1,8-Cineol hinsichtlich der Verbesserung von Ohrenschmerzen sowie des Druck(gefühls) im Ohr ermittelt werden. Weiterhin zeigte die Auswertung der Fragebögen, dass vor Durchführung der Therapie sämtliche Patienten Einschränkungen im Alltag aufwiesen (nämlich 60 % der Patienten große und 40 % der Patienten sehr große Einschränkungen), während nach Therapie eine hohe Zufriedenheit der Patienten im Hinblick auf die Behandlung vorlag. Insbesondere hat sich die Einschränkung im Alltag nach Durchführung der Therapie deutlich verringert, wonach nämlich 90 % der Patienten geringfügige Einschränkungen und nur 10 % der Patienten weiterhin sehr große Einschränkungen angeführt haben.

Zudem wurden in 80 % der Fälle keine Nebenwirkungen angeführt, und kein Patient hat die Einnahme von 1,8-Cineol vorzeitig beendet.

Auch die Auswertung der Patientenfragebögen zeigt somit den erfindungsgemäß aufgezeigten erfolgreichen Therapieansatz auf Basis der Verwendung von 1,8-Cineol im Hinblick auf die zugrundeliegenden Erkrankungen des Ohres.

### Beispiel 6:

### Pathogene Keime vor und nach Therapie mit 1,8-Cineol (Patientenübersicht)

Die Wirksamkeit von 1,8-Cineol im Hinblick auf die Verringerung pathogener Keime bzw. pathogener Bakterien bei Patienten mit chronischer Otitis media wird weiterführend auch auf Basis von Fig. 5 verdeutlicht, welche eine Übersicht über die diesbezüglich untersuchten Patienten zeigt. Insbesondere kann infolge der Bahandlung mit 1,8-Cineol eine Verringerung pathogener Keime bzw. pathogene Bakterien beobachtet werden, und dies sogar auch im Hinblick auf multiresistente pathogener Keime bzw. Bakterien.

### Beispiel 7:

### Pathogene Keime vor und nach Therapie mit 1,8-Cineol - weitere Beispiele mit der Ermöglichung erforderlicher Ohroperationen

Bei den Fig. 6 exemplarisch angeführten drei Patienten (vgl. auch diesbezügliche Patienten gemäß Fig. 5) konnte durch Behandlung mit 1,8-Cineol eine signifikante Reduktion der Entzündung bzw. des Befalls mit pathogenen Keimen erreicht werden, was das operative Einsetzen eines Cochleaimplantats bzw. eines Vibrant-Soundbridge-Implantats ermöglicht hat. Dies führt wiederum zu einer deutlichen Verbesserung des Hörvermögens und damit der Lebensqualität der betroffenen Patienten.

### Beispiel 8:

### Zusammenschau exemplarischer klinischer Krankheits- und Therapieverläufe

Nachfolgend ist eine Zusammenschau exemplarischer klinischer Krankheits- und Therapieverlauf und der erfolgreichen Einflussnahme von 1,8-Cineol in Bezug auf zwei Patienten angeführt.

Die diesbezüglichen Einsatzbereiche sind zum einen chronische Otitis media mesotympanalis (COM) sowie chronische Otitis media epitympanalis (Cholesteatom).

Hinsichtlich des Patienten mit chronischer Otitis media mesotympanalis wurde nach zweiwöchiger peroraler Verabreichung (präoperativ) von dreimal täglich 200 mg 1,8-Cineol bei zuvor therapierefraktärer Otorrhoe (lokale und systemische Antibiotika) ein Rückgang der Otorrhoe beobachtet, so dass hierdurch eine Operation am Ohr ermöglicht worden ist. Bei Zustand nach Tympanoplastik wurde eine erneute Otorrhoe mit Rezidivperforation beobachtet. Nach weiterer Verabreichung von dreimal täglich 200 mg 1,8-Cineol über einen Zeitraum von vier Wochen wurde ein Rückgang auch dieser Otorrhoe und ein spontaner Trommelfellverschluss beobachtet.

Im Hinblick auf den Patienten mit chronischer Otitis media epitympanalis bzw. Cholesteatom wurde nach präoperativer Verabreichung von dreimal täglich 200 mg 1,8-Cineol (Verabreichungsdauer: 2 Wochen, perorale Verabreichung) bei zuvor therapierefraktärer Otorrhoe (lokale und systemische Antibiotika) ein Rückgang der begleitenden Otorrhoe beobachtet, so dass hierdurch eine Operation am Ohr ermöglicht worden ist. Insbesondere lag ein positiver Effekt auf die entzündete Schleimhaut des Mittelohres und im Hinblick auf ein Mastoid vor (wobei zwar die Cholesteatommassen nicht wesentlich reduziert worden sind, jedoch aber - und dies ist therapierelevant - der Entzündungscharakter und die Aggressivität der Entzündung des Cholesteatoms gehemmt bzw. therapiert werden konnte). Bei Zustand nach Tympanoplastik lag eine erneute Otorrhoe mit Rezidivperforation vor, nach Verabreichung von dreimal täglich 200 mg 1,8-Cineol (Verabreichungszeitraum: 4 Wochen, perorale Verabreichung) lag ein Rückgang der Otorrhoe und ein spontaner Trommelfellverschluss auch in diesem Fall vor.

### Beispiel 9:

### Weitere Ausführungen zu Patientendaten und -untersuchungen

In den Industrienationen zählt die Otitis media zu den Erkrankungen, die am häufigsten zu Arztbesuchen, Antibiotikaverschreibungen und Operationen führen. Chronisch rezidivierende Entzündungen des Mittelohres (COM) resultieren in signifikanten Auswirkungen in Bezug auf die Lebensqualität der Patienten z. B. durch Ohrlaufen, Schwerhörigkeit, Sprachentwicklungsverzögerungen etc. bis hin zur völligen Ertaubung und Schwindel. Die besonders aggressive Form, das Cholesteatom (vgl. chronische Otitis Media epitympanalis) verhält sich wie ein bösartiger Tumor, denn es entzieht sich einer Antibiotikatherapie durch fortschreitende entzündlich destruierende Prozesse des Mittelohrs und zerstört die umgebenden Strukturen.

Aus diesem Kollektiv rekrutiert sich das Patientengut, welches im Rahmen der vorliegenden Erfindung präoperativ und auch in Einzelfällen postoperativ mit Soledum^{®} forte-Kapseln behandelt wurde. Die permanente eitrige Otorrhoe war mit konventionellen Mitteln, wie lokalen Ohrentropfen, systemischer Antibiotikatherapie oder verschiedene Spülungen, nicht zu beherrschen. Ein feuchtes Ohr zu operieren, stellt aber den gesamten Operationserfolg in Frage.

Überraschend konnten Otitiden, insbesondere Mittelohrentzündungen, mit chronisch laufendem Ohr erfolgreich mit Soledum^{®} forte-Kapseln behandelt wurde. So wurden Patienten aufgrund einer sehr hartnäckigen Otorrhoe auf Basis einer chronischen Otitis media mesotympanalis oder epitympanalis erfolgreich mit Soledum^{®} forte-Kapseln (dreimal 1 Kapsel je 200 mg 1,8-Cineol täglich) über einen Zeitraum von 14 Tagen behandelt. In allen präoperativen Fällen kam es zu einer deutlichen Reduktion der Otorrhoe, so dass eine zeitnahe Operation möglich wurde und dann auch jeweils zum Erfolg geführt hat. In einigen Fällen wurden Patienten auch erfolgreich postoperativ mit Soledum^{®} forte-Kapseln therapiert.

Ebenfalls erfolgreich verliefen überraschenderweise auch Behandlungen mit Soledum^{®} forte-Kapseln von Patienten mit oft langjähriger chronischer Otits media mesotympanalis mit einhergehender Otorrhoe und kompletter Resistenz im von der Mikrobiologie durchgeführten Antibiogramm auf Erreger vor Therapie.

Insgesamt eignet sich somit Cineol, insbesondere 1,8-Cineol, auf Basis der überraschend gefundenen Erkenntnisse der Anmelderin in effizienter Weise zur Verwendung bei der prophylaktischen bzw. therapeutischen Behandlung von entzündlichen Erkrankungen des Ohres, vorzugsweise des Mittelohres, mit begleitender bzw. einhergehender Otorrhoe, bzw. zur Verwendung bei der prophylaktischen bzw. therapeutischen Behandlung von Otitis media mit begleitender Otorrhoe.

## Patentansprüche

1. Cineol, vorzugsweise 1,8-Cineol, zur Verwendung bei der prophylaktischen und/oder therapeutischen Behandlung von Otitis media mit begleitender Otorrhoe,
wobei das Cineol systemisch appliziert wird,
wobei das Cineol als Reinstoff vorliegt und wobei das Cineol frei von anderen Terpenen ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

2. Cineol, vorzugsweise 1,8-Cineol, zur Verwendung nach Anspruch 1,
zur Verwendung bei der Reduktion der Last und/oder der Anzahl von im Mittelohr befindlichen und/oder lokalisierten pathogenen Keimen, insbesondere pathogenen Bakterien,
bei der prophylaktischen und/oder therapeutischen Behandlung von Otitis media mit begleitender Otorrhoe.

3. Cineol, vorzugsweise 1,8-Cineol, zur Verwendung nach Anspruch 1 oder 2,
wobei das Cineol, vorzugsweise 1,8-Cineol, zur Reduktion der Last und/oder der Anzahl von im Mittelohr befindlichen und/oder lokalisierten pathogenen Keimen, insbesondere pathogenen Bakterien, eingesetzt oder verwendet wird; und/oder
wobei die pathogenen Keime ausgewählt sind aus der Gruppe von die Otitis media mit begleitender Otorrhoe verursachenden und/oder verstärkenden Bakterien; und/oder
wobei die pathogenen Keime ausgewählt sind aus der Gruppe von Bakterien der Gattung *Pseudomonas, Proteus, Staphylococcus, Haemophilus, Streptococcus, Stenotrophomonas, Enterococcus* und *Moraxella* sowie deren Kombinationen, insbesondere aus der Gruppe von Bakterien der Gattung *Pseudomonas, Proteus* und *Staphylococcus* sowie deren Kombinationen, vorzugsweise aus der Gruppe von Bakterien der Gattung *Pseudomonas* und *Proteus* sowie deren Kombinationen; und/oder
wobei die pathogenen Keime ausgewählt sind aus der Gruppe von Bakterien der Spezies *Pseudomonas aeruginosa, Pseudomonas stutzeri, Proteus mirabilis, Staphylococcus aureus, Haemophilus influenza, Streptococcus pneumoniae, Streptococcus pyogenes, Stenotrophomonas maltophila, Enterococcus faecalis* und *Moraxella catarrhalis* sowie deren Kombinationen, insbesondere aus der Gruppe von Bakterien der Spezies *Pseudomonas aeruginosa, Proteus mirabilis* und *Staphylococcus aureus* sowie deren Kombinationen, vorzugsweise von Bakterien der Spezies *Pseudomonas aeruginosa* und *Proteus mirabilis* sowie deren Kombinationen.

4. Cineol, vorzugsweise 1,8-Cineol, zur Verwendung nach einem der vorangehenden Ansprüche,
wobei die Otitis eine therapierefraktäre und/oder therapieresistente, insbesondere medikamenten- und/oder arzneimittelresistente, vorzugsweise antibiotikaresistente, Otitis ist; und/oder
wobei die Otitis eine akute Otitis oder chronische Otitis, insbesondere eine chronische Otitis, vorzugsweise eine chronisch polypöse Otitis oder eine chronisch granuläre Otitis, ist; und/oder
wobei die Otitis eine rezidivierende Otitis oder chronisch-rezidivierende Otitis, insbesondere eine chronische Otitis, vorzugsweise eine chronisch polypöse Otitis oder eine chronisch granuläre Otitis, ist; und/oder
wobei die Otitis eine Otitis media epitympanalis oder eine Otitis media mesotympanalis, vorzugsweise eine Otitis media epitympanalis, ist; und/oder
wobei die Otitis media mit begleitender Otorrhoe, bevorzugt Otitis media epitympanalis, mit einer Entzündungsmediation, insbesondere einer Heraufregulation und/oder einer erhöhten Produktion oder einer erhöhten Freisetzung von insbesondere proinflammatorischen Zytokinen, vorzugsweise TNF-alpha und/oder Interleukin-1, bevorzugt TNF-alpha, einhergeht oder hiermit im Zusammenhang steht.

5. Cineol, vorzugsweise 1,8-Cineol, zur Verwendung nach einem der vorangehenden Ansprüche,
wobei das Cineol, vorzugsweise 1,8-Cineol, zusätzlich und/oder gleichermaßen zur Suppression oder Abschwächung einer im Rahmen Otitis media mit begleitender Otorrhoe, bevorzugt Otitis media epitympanalis, auftretenden Entzündungsmediation verwendet oder eingesetzt wird; und/oder
wobei das Cineol, vorzugsweise 1,8-Cineol, eine supprimierende oder abschwächende Wirkung gegenüber einer im Rahmen der Otitis media mit begleitender Otorrhoe, bevorzugt Otitis media epitympanalis, auftretenden Entzündungsmediation ausübt, insbesondere wobei das Cineol, vorzugsweise 1,8-Cineol, eine Herabregulation und/oder eine verringerte Produktion und/oder eine verringerte Ausschüttung von insbesondere proinflammatorischen Zytokinen, vorzugsweise TNF-alpha und/oder Interleukin-1beta, bevorzugt TNF-alpha, bewirkt und/oder hervorruft; und/oder
wobei das Cineol, vorzugsweise 1,8-Cineol, zur Herabregulation und/oder zur Verringerung der Produktion und/oder zur Verringerung der Ausschüttung von insbesondere proinflammatorischen Zytokinen, vorzugsweise TNF-alpha und/oder Interleukin-1beta, bevorzugt TNF-alpha, verwendet oder eingesetzt wird.

6. Cineol, vorzugsweise 1,8-Cineol, zur Verwendung nach einem der vorangehenden Ansprüche,
wobei das Cineol, vorzugsweise 1,8-Cineol, zusätzlich und/oder gleichermaßen als antiinflammatorischer Wirkstoff, insbesondere als lokal und/oder systemisch wirkender antiinflammatorischer Wirkstoff, verwendet oder eingesetzt wird; und/oder
wobei das Cineol, vorzugsweise 1,8-Cineol, zusätzlich und/oder gleichermaßen zur Vorbeugung und/oder Verhinderung und/oder zur prophylaktischen und/oder therapeutischen Behandlung von mit der Otitis media mit begleitender Otorrhoe im Zusammenhang stehenden und/oder hierdurch hervorgerufenen Folge- und/oder Begleiterkrankungen, insbesondere ausgewählt aus der Gruppe von Mastoisitis, Labyrinthitis, Hirnhautentzündung, Sinusvenenthrombose, Sepsis, Gesichtsnervenlähmung und Tinnitus sowie deren Kombinationen, verwendet oder eingesetzt wird.

7. Cineol, vorzugsweise 1,8-Cineol, zur Verwendung nach einem der vorangehenden Ansprüche,
wobei das Cineol, vorzugsweise 1,8-Cineol, präoperativ und/oder zur präoperativen Vorbereitung und/oder zur Ermöglichung von chirurgischen Eingriffen, insbesondere therapeutisch-chirurgischen Eingriffen, am Ohr, wie tympanoplastischen Operationen und/oder Implantationsoperationen, verwendet oder eingesetzt wird; und/oder
wobei das Cineol, vorzugsweise 1,8-Cineol, postoperativ und/oder zur postoperativen Nachbehandlung von chirurgischen Eingriffen, insbesondere therapeutisch-chirurgischen Eingriffen, am Ohr, wie tympanoplastischen Operationen und/oder Implantationsoperationen, verwendet oder eingesetzt wird.

8. Cineol, vorzugsweise 1,8-Cineol, zur Verwendung nach einem der vorangehenden Ansprüche,
jedoch ausgenommen zur Verwendung bei der Verringerung pathogener Keime bzw. pathogenen Erregern im menschlichen Darm, insbesondere in der menschlichen Darmflora, und/oder jedoch ausgenommen zur Verwendung bei der Reduktion der Besiedlung des menschlichen Darms, insbesondere der menschlichen Darmflora, mit pathogenen Keimen bzw. pathogenen Erregern.

9. Cineol, vorzugsweise 1,8-Cineol, zur Verwendung nach einem der vorangehenden Ansprüche,
wobei das Cineol, vorzugsweise 1,8-Cineol, peroral oder parenteral, vorzugsweise peroral, appliziert wird; und/oder
wobei das Cineol, vorzugsweise 1,8-Cineol, in Form einer peroral zu verabreichenden Darreichungsform appliziert wird; und/oder
wobei das Cineol, vorzugsweise 1,8-Cineol, als magensaftresistente, aber dünndarmlösliche systemische Darreichungsform, bevorzugt als Kapsel, Dragee, Pille, Tablette oder dergleichen, appliziert wird.

10. Cineol, vorzugsweise 1,8-Cineol, zur Verwendung nach einem der vorangehenden Ansprüche,
wobei das Cineol, vorzugsweise 1,8-Cineol, mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, vorzugsweise 1,8-Cineol, vorliegt.

11. Cineol, vorzugsweise 1,8-Cineol, zur Verwendung nach einem der Ansprüche 1 bis 10,
wobei das Cineol, vorzugsweise 1,8-Cineol, als Einzelwirkstoff und/oder als Monopräparat vorliegt; und/oder
wobei das Cineol, vorzugsweise 1,8-Cineol, ohne und/oder in Abwesenheit von weiteren, von Cineol, vorzugsweise 1,8-Cineol, verschiedenen Wirkstoffen vorliegt; und/oder
wobei das Cineol, vorzugsweise 1,8-Cineol, ohne und/oder in Abwesenheit von und/oder frei von und/oder nicht in Kombination mit Vitamin D-Rezeptoragonisten, insbesondere Verbindungen der Vitamin D-Gruppe, vorliegt.

12. Cineol, vorzugsweise 1,8-Cineol, zur Verwendung nach einem der Ansprüche 1 bis 10,
wobei das Cineol, vorzugsweise 1,8-Cineol, zusammen mit mindestens einem weiteren Wirkstoff und/oder als Co-Therapeutikum vorliegt;
insbesondere wobei der weitere Wirkstoff ausgewählt ist aus (i) entzündungshemmenden Wirkstoffen, insbesondere nichtsteroidalen Entzündungshemmern oder steroidalen Entzündungshemmern; (ii) Analgetika; (iii) Antipyretika; (iv) Antibiotika; sowie deren Kombinationen; und/oder
insbesondere wobei der weitere Wirkstoff getrennt, insbesondere räumlich getrennt, von dem Cineol, vorzugsweise 1,8-Cineol, aber funktional zusammenhängend hiermit eingesetzt wird, insbesondere in Form eines Kit.

13. Arzneimittel oder Medikament, insbesondere Ohrbehandlungsmittel, zur Verwendung bei der prophylaktischen und/oder therapeutischen Behandlung von Otitis media mit begleitender Otorrhoe,
wobei das Arzneimittel oder Medikament Cineol, vorzugsweise 1,8-Cineol, zusammen mit mindestens einem pharmazeutisch verträglichen und/oder physiologisch unbedenklichen Exzipienten enthält,
wobei das Arzneimittel oder Medikament systemisch appliziert wird,
wobei das Arzneimittel oder Medikament das Cineol als Reinstoff enthält und wobei das Arzneimittel oder Medikament frei von anderen Terpenen als Cineol ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

14. Zusammensetzung, insbesondere pharmazeutische Zusammensetzung, zur Verwendung bei der prophylaktischen und/oder therapeutischen Behandlung von Otitis media mit begleitender Otorrhoe,
wobei die Zusammensetzung Cineol, vorzugsweise 1,8-Cineol, insbesondere zusammen mit mindestens einem pharmazeutisch verträglichen und/oder physiologisch unbedenklichen Exzipienten, enthält,
wobei die Zusammensetzung systemisch appliziert wird,
wobei die Zusammensetzung das Cineol als Reinstoff enthält und wobei die Zusammensetzung frei von anderen Terpenen als Cineol ist;
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird.

15. Pharmazeutische Kombination zur Verwendung bei der prophylaktischen und/oder therapeutischen Behandlung von Otitis media mit begleitender Otorrhoe,
wobei die pharmazeutische Kombination mindestens die nachfolgenden Komponenten (A) und (B) umfasst, wobei die Komponenten (A) und (B) getrennt voneinander, aber funktional zusammenhängend und/oder funktional einander zugeordnet vorliegen und/oder verabreicht werden:
(A) Cineol, vorzugsweise 1,8-Cineol,
wobei das Cineol systemisch appliziert wird,
wobei das Cineol als Reinstoff vorliegt und wobei das Cineol frei von anderen Terpenen ist,
wobei das Cineol des Weiteren und/oder zusätzlich und/oder gleichermaßen zur Erhöhung der Anzahl, Vermehrung oder Unterstützung von im Ohr, insbesondere im Mittel- und/oder Außenohr, vorzugsweise im Mittelohr, befindlichen und/oder lokalisierten physiologischen Keimen verwendet oder eingesetzt wird; sowie
(B) mindestens einen weiteren Wirkstoff, ausgewählt aus (i) entzündungshemmenden Wirkstoffen, insbesondere nichtsteroidalen Entzündungshemmern oder steroidalen Entzündungshemmern; (ii) Analgetika; (iii) Antipyretika; (iv) Antibiotika; sowie deren Kombinationen.

## Claims

1. Cineole, preferably 1,8-cineole, for use in the prophylactic and/or therapeutic treatment of otitis media with concomitant otorrhea,
wherein the cineole is applied systemically,
wherein the cineole is present as a pure substance and wherein the cineole is free of other terpenes;
wherein the cineole is further and/or additionally and/or equally used or employed to increase the quantity, multiplication or support of physiological germs located and/or localized in the ear, especially in the middle and/or outer ear, preferably in the middle ear.

2. Cineole, preferably 1,8-cineole, for use according to claim 1,
for use in the reduction of the load and/or quantity of pathogenic germs, especially pathogenic bacteria, located and/or localized in the middle ear,
in the prophylactic and/or therapeutic treatment of otitis media with concomitant otorrhea.

3. Cineole, preferably 1,8-cineole, for use according to claim 1 or 2,
wherein the cineole, preferably 1,8-cineole, is employed or used to reduce the load and/or the quantity of pathogenic germs, especially pathogenic bacteria, located and/or localized in the middle ear; and/or
wherein the pathogenic germs are selected from the group of bacteria causing and/or aggravating otitis media with concomitant otorrhea; and/or
wherein the pathogenic germs are selected from the group of bacteria of the genus *Pseudomonas, Proteus, Staphylococcus, Haemophilus, Streptococcus, Stenotrophomonas, Enterococcus* and *Moraxella* as well as combinations thereof, especially from the group of bacteria of the genus *Pseudomonas, Proteus* and *Staphylococcus* as well as combinations thereof, preferably from the group of bacteria of the genus *Pseudomonas* and *Proteus* as well as combinations thereof; and/or
wherein the pathogenic germs are selected from the group of bacteria of the species *Pseudomonas aeruginosa, Pseudomonas stutzeri, Proteus mirabilis, Staphylococcus aureus, Haemophilus influenza, Streptococcus pneumoniae, Streptococcus pyogenes, Stenotrophomonas maltophila, Enterococcus faecalis* and *Moraxella catarrhalis* as well as combinations thereof, especially from the group of bacteria of the species *Pseudomonas aeruginosa, Proteus mirabilis* and *Staphylococcus aureus* as well as combinations thereof, preferably from bacteria of the species *Pseudomonas aeruginosa* and *Proteus mirabilis* as well as combinations thereof.

4. Cineole, preferably 1,8-cineole, for use according to any of the preceding claims,
wherein the otitis is a therapy-refractory and/or therapy-resistant, especially drug-resistant and/or pharmaceutical-resistant, preferably antibiotic-resistant, otitis; and/or
wherein the otitis is an acute otitis or chronic otitis, especially a chronic otitis, preferably a chronic polypous otitis or a chronic granular otitis; and/or
wherein the otitis is a recurrent otitis or chronic recurrent otitis, especially a chronic otitis, preferably a chronic polypous otitis or a chronic granular otitis; and/or
wherein the otitis is an otitis media epitympanalis or an otitis media mesotympanalis, preferably an otitis media epitympanalis; and/or
wherein the otitis media with concomitant otorrhea, preferably otitis media epitympanalis, is accompanied by or associated with inflammatory mediation, especially upregulation and/or increased production or increased release of especially proinflammatory cytokines, preferably TNF-alpha and/or interleukin-1, preferably TNF-alpha.

5. Cineole, preferably 1,8-cineole, for use according to any of the preceding claims,
wherein the cineole, preferably 1,8-cineole, is additionally and/or equally used or applied to suppress or attenuate inflammatory mediation occurring in the context of otitis media with concomitant otorrhea, preferably otitis media epitympanalis; and/or
wherein the cineole, preferably 1,8-cineole, exerts a suppressive or alleviating effect against inflammatory mediation occurring in the context of otitis media with concomitant otorrhea, preferably otitis media epitympanalis, especially wherein the cineole, preferably 1,8-cineole, causes and/or induces a downregulation and/or a reduced production and/or a reduced release of especially proinflammatory cytokines, preferably TNF-alpha and/or interleukin-1beta, preferably TNF-alpha; and/or
wherein the cineole, preferably 1,8-cineole, is used or employed to down-regulate and/or to reduce the production and/or to reduce the release of especially pro-inflammatory cytokines, preferably TNF-alpha and/or interleukin-1beta, preferably TNF-alpha.

6. Cineole, preferably 1,8-cineole, for use according to any of the preceding claims,
wherein the cineole, preferably 1,8-cineole, is additionally and/or equally used or employed as an anti-inflammatory agent, especially as a locally and/or systemically acting anti-inflammatory agent; and/or
wherein the cineole, preferably 1,8-cineole, is additionally and/or equally used or employed for the prevention and/or avoidance and/or prophylactic and/or therapeutic treatment of secondary and/or concomitant diseases associated with and/or caused by otitis media with concomitant otorrhea, especially selected from the group of mastoisitis, labyrinthitis, meningitis, sinus vein thrombosis, sepsis, facial nerve paralysis and tinnitus as well as combinations thereof.

7. Cineole, preferably 1,8-cineole, for use according to any of the preceding claims,
wherein the cineole, preferably 1,8-cineole, is used or employed preoperatively and/or for preoperative preparation and/or to enable surgical interventions, especially therapeutic surgical interventions, on the ear, such as tympanoplasty operations and/or implantation operations; and/or
wherein the cineole, preferably 1,8-cineole, is used or employed postoperatively and/or for postoperative follow-up treatment of surgical interventions, especially therapeutic surgical interventions, on the ear, such as tympanoplastic operations and/or implantation operations.

8. Cineole, preferably 1,8-cineole, for use according to any of the preceding claims,
however, excluding for use in the reduction of pathogenic germs or pathogenic agents in the human intestine, especially in the human intestinal flora, and/or however, excluding for use in the reduction of the colonization of the human intestine, especially of the human intestinal flora, with pathogenic germs or pathogenic agents.

9. Cineole, preferably 1,8-cineole, for use according to any of the preceding claims,
wherein the cineole, preferably 1,8-cineole, is administered perorally or parenterally, preferably perorally; and/or
wherein the cineole, preferably 1,8-cineole, is applied in the form of a dosage form to be administered perorally; and/or
wherein the cineole, preferably 1,8-cineole, is administered as an enteric-coated but small intestine-soluble systemic dosage form, preferably as a capsule, coated tablet, pill, tablet or the like.

10. Cineole, preferably 1,8-cineole, for use according to any of the preceding claims,
wherein the cineole, preferably 1,8-cineole, is present with a purity of at least 95 % by weight, especially at least 96 % by weight, preferably at least 97 % by weight, more preferably at least 98 % by weight, even more preferably at least 99 % by weight, still more preferably at least 99.5 %, based on the cineole, preferably 1,8-cineole.

11. Cineole, preferably 1,8-cineole, for use according to any one of claims 1 to 10,
wherein the cineole, preferably 1,8-cineole, is present as a single active ingredient and/or as a monopreparation; and/or
wherein the cineole, preferably 1,8-cineole, is present without and/or in the absence of further active ingredients other than cineole, preferably 1,8-cineole; and/or
wherein the cineole, preferably 1,8-cineole, is present without and/or in the absence of and/or free of and/or not in combination with vitamin D receptor agonists, especially compounds of the vitamin D group.

12. Cineole, preferably 1,8-cineole, for use according to any one of claims 1 to 10,
wherein the cineole, preferably 1,8-cineole, is present together with at least one further active ingredient and/or as a co-therapeutic agent;
especially wherein the further active ingredient is selected from (i) anti-inflammatory agents, especially non-steroidal anti-inflammatory agents or steroidal anti-inflammatory agents; (ii) analgesics; (iii) antipyretics; (iv) antibiotics; as well as combinations thereof; and/or
especially wherein the further active ingredient is used separately, especially spatially separated, from the cineole, preferably 1,8-cineole, but functionally coherent therewith, especially in the form of a kit.

13. Drug or medicament, especially ear treatment composition, for use in the prophylactic and/or therapeutic treatment of otitis media with concomitant otorrhea,
wherein the drug or medicament contains cineole, preferably 1,8-cineole, together with at least one pharmaceutically acceptable and/or physiologically acceptable excipient,
wherein the drug or medication is applied systemically,
wherein the drug or medicament contains the cineole as a pure substance and wherein the drug or medicament is free from terpenes other than cineole;
wherein the cineole is further and/or additionally and/or equally used or employed to increase the quantity, multiplication or support of physiological germs located and/or localized in the ear, especially in the middle and/or outer ear, preferably in the middle ear.

14. A composition, especially a pharmaceutical composition, for use in the prophylactic and/or therapeutic treatment of otitis media with concomitant otorrhea,
wherein the composition contains cineole, preferably 1,8-cineole, especially together with at least one pharmaceutically acceptable and/or physiologically acceptable excipient,
wherein the composition is applied systemically,
wherein the composition contains the cineole as a pure substance and wherein the composition is free of terpenes other than cineole;
wherein the cineole is further and/or additionally and/or equally used or employed to increase the quantity, multiplication or support of physiological germs located and/or localized in the ear, especially in the middle and/or outer ear, preferably in the middle ear.

15. Pharmaceutical combination for use in the prophylactic and/or therapeutic treatment of otitis media with concomitant otorrhea,
wherein the pharmaceutical combination comprises at least the following components (A) and (B), wherein the components (A) and (B) are present and/or administered independent from one another, but functionally coherent and/or functionally associated with each other:
(A) cineole, preferably 1,8-cineole,
wherein the cineole is applied systemically,
wherein the cineole is present as a pure substance and wherein the cineole is free of other terpenes,
wherein the cineole is further and/or additionally and/or equally used or employed to increase the quantity, multiplication or support of physiological germs located and/or localized in the ear, especially in the middle and/or outer ear, preferably in the middle ear; and
(B) at least one further active ingredient selected from (i) anti-inflammatory active ingredients, especially non-steroidal anti-inflammatory agents or steroidal anti-inflammatory agents; (ii) analgesics; (iii) antipyretics; (iv) antibiotics; as well as combinations thereof.

## Revendications

1. Cinéole, de préférence 1,8-cinéole, à utiliser dans le traitement prophylactique et/ou thérapeutique de l'otite moyenne accompagnée d'otorrhée,
le cinéole étant appliqué par voie systémique,
où le cinéol est présent sous forme de substance pure et où le cinéol est exempt d'autres terpènes;
le cinéole étant en outre et/ou en plus et/ou de la même manière utilisé ou mis en oeuvre pour augmenter le nombre, la multiplication ou le soutien de germes physiologiques se trouvant et/ou étant localisés dans l'oreille, en particulier dans l'oreille moyenne et/ou l'oreille externe, de préférence dans l'oreille moyenne.

2. Cinéole, de préférence 1,8-cinéole, pour l'utilisation selon la revendication 1,
à utiliser pour réduire la charge et/ou le nombre de germes pathogènes, en particulier de bactéries pathogènes, présents et/ou localisés dans l'oreille moyenne,
dans le traitement prophylactique et/ou thérapeutique de l'otite moyenne avec otorrhée concomitante.

3. Cinéole, de préférence 1,8-cinéole, pour l'utilisation selon la revendication 1 ou 2,
le cinéole, de préférence le 1,8-cinéole, étant mis en oeuvre ou utilisé pour réduire la charge et/ou le nombre de germes pathogènes, en particulier de bactéries pathogènes, présents et/ou localisés dans l'oreille moyenne; et/ou
les germes pathogènes étant choisis dans le groupe des bactéries provoquant et/ou aggravant l'otite moyenne avec otorrhée concomitante; et/ou
les germes pathogènes étant choisis parmi le groupe de bactéries du genre *Pseudomonas, Proteus, Staphylococcus, Haemophilus, Streptococcus, Stenotrophomonas, Enterococcus* et *Moraxella* ainsi que leurs combinaisons, en particulier parmi le groupe de bactéries du genre *Pseudomonas, Proteus* et *Staphylococcus* ainsi que leurs combinaisons, de préférence parmi le groupe de bactéries du genre *Pseudomonas* et *Proteus* ainsi que leurs combinaisons; et/ou
les germes pathogènes étant choisis dans le groupe de bactéries des espèces *Pseudomonas aeruginosa, Pseudomonas stutzeri, Proteus mirabilis, Staphylococcus aureus, Haemophilus influenza, Streptococcus pneumoniae, Streptococcus pyogenes, Stenotrophomonas maltophila, Enterococcus faecalis* et *Moraxella catarrhalis* ainsi que leurs combinaisons, en particulier parmi le groupe de bactéries des espèces *Pseudomonas aeruginosa, Proteus mirabilis* et *Staphylococcus aureus* ainsi que leurs combinaisons, de préférence parmi les bactéries des espèces *Pseudomonas aeruginosa* et *Proteus mirabilis* ainsi que leurs combinaisons.

4. Cinéole, de préférence 1,8-cinéole, pour utilisation selon l'une quelconque des revendications précédentes,
l'otite étant une otite réfractaire et/ou résistante au traitement, en particulier résistante aux médicaments et/ou aux produits pharmaceutiques, de préférence résistante aux antibiotiques; et/ou
l'otite étant une otite aiguë ou une otite chronique, en particulier une otite chronique, de préférence une otite polypeuse chronique ou une otite granuleuse chronique; et/ou
l'otite étant une otite récidivante ou une otite chronique récidivante, en particulier une otite chronique, de préférence une otite chronique polypoïde ou une otite chronique granuleuse; et/ou
l'otite étant une otite moyenne épitympanique ou une otite moyenne mésotympanique, de préférence une otite moyenne épitympanique; et/ou
l'otite moyenne avec otorrhée concomitante, de préférence otite moyenne épitympanique, étant associée à une médiation inflammatoire, en particulier à une régulation à la hausse et/ou à une production accrue ou à une libération accrue de cytokines en particulier pro-inflammatoires, de préférence TNF-alpha et/ou interleukine-1, de préférence TNF-alpha, ou étant en relation avec celles-ci.

5. Cinéole, de préférence 1,8-cinéole, pour utilisation selon l'une quelconque des revendications précédentes,
le cinéole, de préférence le 1,8-cinéole, étant utilisé ou mis en oeuvre en plus et/ou de manière identique pour supprimer ou atténuer une médiation inflammatoire se produisant dans le cadre d'une otite moyenne avec otorrhée concomitante, de préférence une otite moyenne épitympanique; et/ou
le cinéole, de préférence le 1,8-cinéole, exerçant un effet supprimant ou atténuant vis-à-vis d'une médiation inflammatoire survenant dans le cadre de l'otite moyenne avec otorrhée concomitante, de préférence l'otite moyenne épitympanique, en particulier le cinéole, de préférence le 1,8-cinéole, provoque et/ou entraîne une diminution de la régulation et/ou de la production et/ou de la libération de cytokines notamment pro-inflammatoires, de préférence le TNF-alpha et/ou l'interleukine-1bêta, de préférence le TNF-alpha; et/ou
le cinéol, de préférence le 1,8-cinéol, étant utilisé ou mis en oeuvre pour abaisser la régulation et/ou pour réduire la production et/ou pour réduire la sécrétion de cytokines en particulier pro-inflammatoires, de préférence le TNF-alpha et/ou l'interleukine-1bêta, de préférence le TNF-alpha.

6. Cinéole, de préférence 1,8-cinéole, pour utilisation selon l'une quelconque des revendications précédentes,
le cinéol, de préférence le 1,8-cinéol, étant utilisé ou mis en oeuvre en plus et/ou de manière identique comme substance active anti-inflammatoire, en particulier comme substance active anti-inflammatoire à action locale et/ou systémique; et/ou
le cinéole, de préférence le 1,8-cinéole, étant utilisé ou mis en oeuvre en plus et/ou de manière identique pour la prévention et/ou l'empêchement et/ou pour le traitement prophylactique et/ou thérapeutique de maladies consécutives et/ou concomitantes liées à l'otite moyenne avec otorrhée associée et/ou provoquées par celle-ci, en particulier choisies dans le groupe de la mastoïdite, de la labyrinthite, de la méningite, de la thrombose des sinus veineux, de la septicémie, de la paralysie des nerfs faciaux et des acouphènes ainsi que de leurs combinaisons.

7. Cinéole, de préférence 1,8-cinéole, pour utilisation selon l'une quelconque des revendications précédentes,
le cinéole, de préférence le 1,8-cinéole, étant utilisé ou mis en oeuvre de manière préopératoire et/ou pour la préparation préopératoire et/ou pour permettre des interventions chirurgicales, en particulier des interventions de chirurgie thérapeutique, sur l'oreille, telles que des opérations de tympanoplastie et/ou des opérations d'implantation; et/ou
le cinéole, de préférence le 1,8-cinéole, étant utilisé ou mis en oeuvre en postopératoire et/ou pour le traitement postopératoire postopératoire d'interventions chirurgicales, en particulier d'interventions chirurgicales thérapeutiques, sur l'oreille, telles que des opérations de tympanoplastie et/ou des opérations d'implantation.

8. Cinéole, de préférence 1,8-cinéole, pour utilisation selon l'une quelconque des revendications précédentes,
mais à l'exclusion de l'utilisation pour la réduction de germes pathogènes ou d'agents pathogènes dans l'intestin humain, en particulier dans la flore intestinale humaine, et/ou à l'exclusion de l'utilisation pour la réduction de la colonisation de l'intestin humain, en particulier de la flore intestinale humaine, par des germes pathogènes ou des agents pathogènes.

9. Cinéole, de préférence 1,8-cinéole, pour utilisation selon l'une quelconque des revendications précédentes,
le cinéol, de préférence le 1,8-cinéol, étant appliqué par voie perorale ou parentérale, de préférence par voie perorale; et/ou
le cinéol, de préférence le 1,8-cinéol, étant appliqué sous la forme d'une présentation à administrer par voie perorale; et/ou
le cinéol, de préférence le 1,8-cinéol, étant appliqué sous une forme d'administration systémique résistante au suc gastrique mais soluble dans l'intestin grêle, de préférence sous forme de capsule, de dragée, de pilule, de comprimé ou similaire.

10. Cinéole, de préférence 1,8-cinéole, pour utilisation selon l'une quelconque des revendications précédentes,
le cinéol, de préférence le 1,8-cinéol, étant présent avec une pureté d'au moins 95 % en poids, en particulier d'au moins 96 % en poids, de préférence d'au moins 97 % en poids, de manière particulièrement préférée d'au moins 98 % en poids, de manière tout particulièrement préférée d'au moins 99 % en poids, de manière encore plus préférée d'au moins 99,5 %, par rapport au cinéol, de préférence 1,8-cinéol.

11. Cinéole, de préférence 1,8-cinéole, pour une utilisation selon l'une quelconque des revendications 1 à 10,
le cinéol, de préférence le 1,8-cinéol, étant présent sous forme de substance active unique et/ou de monopréparation; et/ou
le cinéol, de préférence le 1,8-cinéol, étant présent sans et/ou en l'absence d'autres substances actives différentes du cinéol, de préférence du 1,8-cinéol; et/ou
le cinéol, de préférence le 1,8-cinéol, étant présent sans et/ou en l'absence de et/ou exempt de et/ou non en combinaison avec des agonistes des récepteurs de la vitamine D, en particulier des composés du groupe de la vitamine D.

12. Cinéole, de préférence 1,8-cinéole, pour utilisation selon l'une quelconque des revendications 1 à 10,
le cinéol, de préférence le 1,8-cinéol, étant présent conjointement avec au moins un autre principe actif et/ou en tant que co-thérapeutique;
en particulier où l'autre substance active est choisie parmi (i) des substances actives anti-inflammatoires, en particulier des anti-inflammatoires non stéroïdiens ou des anti-inflammatoires stéroïdiens; (ii) des analgésiques; (iii) des antipyrétiques; (iv) des antibiotiques; ainsi que leurs combinaisons; et/ou
en particulier où l'autre substance active est utilisée séparément, en particulier spatialement séparée, du cinéol, de préférence du 1,8-cinéol, mais fonctionnellement liée à celui-ci, en particulier sous la forme d'un kit.

13. Médicament ou médicament, en particulier produit de traitement de l'oreille, destiné à être utilisé dans le traitement prophylactique et/ou thérapeutique de l'otite moyenne avec otorrhée concomitante,
le médicament ou le remède contenant du cinéol, de préférence du 1,8-cinéol, conjointement avec au moins un excipient pharmaceutiquement acceptable et/ou physiologiquement acceptable,
où le médicament ou le produit pharmaceutique est appliqué par voie systémique,
où le médicament ou le remède contient le cinéol sous forme de substance pure et où le médicament ou le remède est exempt de terpènes autres que le cinéol;
le cinéole étant en outre et/ou en plus et/ou de la même manière utilisé ou mis en oeuvre pour augmenter le nombre, la multiplication ou le soutien de germes physiologiques se trouvant et/ou localisés dans l'oreille, en particulier dans l'oreille moyenne et/ou l'oreille externe, de préférence dans l'oreille moyenne.

14. Composition, en particulier composition pharmaceutique, destinée à être utilisée dans le traitement prophylactique et/ou thérapeutique de l'otite moyenne avec otorrhée concomitante,
ladite composition contenant du cinéol, de préférence du 1,8-cinéol, en particulier conjointement avec au moins un excipient pharmaceutiquement acceptable et/ou physiologiquement acceptable,
la composition étant appliquée par voie systémique,
où la composition contient le cinéol sous forme de substance pure et où la composition est exempte de terpènes autres que le cinéol;
le cinéole étant en outre et/ou en plus et/ou de la même manière utilisé ou mis en oeuvre pour augmenter le nombre, la multiplication ou le soutien de germes physiologiques se trouvant et/ou localisés dans l'oreille, en particulier dans l'oreille moyenne et/ou l'oreille externe, de préférence dans l'oreille moyenne.

15. Combinaison pharmaceutique destinée à être utilisée dans le traitement prophylactique et/ou thérapeutique de l'otite moyenne avec otorrhée concomitante,
la combinaison pharmaceutique comprenant au moins les composants (A) et (B) suivants, les composants (A) et (B) étant présents et/ou administrés séparément l'un de l'autre, mais fonctionnellement liés et/ou fonctionnellement associés l'un à l'autre:
(A) Cinéole, de préférence 1,8-cinéole,
le cinéole étant appliqué par voie systémique,
où le cinéol est présent sous forme de substance pure et où le cinéol est exempt d'autres terpènes,
le cinéole étant en outre et/ou en plus et/ou de la même manière utilisé ou mis en oeuvre pour augmenter le nombre, la multiplication ou le soutien de germes physiologiques se trouvant et/ou localisés dans l'oreille, en particulier dans l'oreille moyenne et/ou l'oreille externe, de préférence dans l'oreille moyenne; ainsi que
(B) au moins une autre substance active choisie parmi (i) les substances actives anti-inflammatoires, en particulier les anti-inflammatoires non stéroïdiens ou les anti-inflammatoires stéroïdiens; (ii) les analgésiques; (iii) les antipyrétiques; (iv) les antibiotiques; ainsi que leurs combinaisons.
